# EUROPEAN PATENT APPLICATION

(11) **EP 4 353 251 A1**
(43) Date of publication of application: **17.04.2024**
(21) Application number: 22804668.6
(22) Date of filing: 17.05.2022
(51) Int. Cl.: A61K 38/44, A61K 45/00, A61K 47/54, A61P 1/16, A61P 13/12, A61P 25/00, A61P 27/16, A61P 35/00, A61P 39/00

(54) **PHARMACEUTICAL COMPOSITION FOR TREATING OR PREVENTING DISORDER ASSOCIATED WITH ADMINISTRATION OF ANTICANCER AGENT**

(30) Priority: 18.05.2021 JP 2021083610
(71) Applicant: LTT Bio-Pharma Co., Ltd., Minato-ku Tokyo 105-0022 (JP); Musashino University, Tokyo 135-8181 (JP)
(72) Inventor: QIAO, Zhiwei, Fujisawa-shi, Kanagawa 251-8555 (JP); HONDA, Manami, Fujisawa-shi, Kanagawa 251-8555 (JP); AKIMOTO, Shouta, Fujisawa-shi, Kanagawa 251-8555 (JP); SASAKI, Tomoki, Fujisawa-shi, Kanagawa 251-8555 (JP); KAJI, Noriko, Fujisawa-shi, Kanagawa 251-8555 (JP); FUKUDA, Koichiro, Tokyo 105-0022 (JP); MIZUSHIMA, Tohru, Tokyo 105-0022 (JP); TANAKA, Ken-ichiro, Nishitokyo-shi, Tokyo 202-8585 (JP)
(74) Representative: Wächtershäuser & Hartz Patentanwaltspartnerschaft mbB
(86) International application number: PCT/JP2022/020473
(87) International publication number: WO 2022/244757

(57) **Abstract**

A pharmaceutical composition for treating or preventing a disorder associated with administration of an anticancer agent. The present invention relates to a pharmaceutical composition for treating or preventing a disorder associated with administration of an anticancer agent, the pharmaceutical composition comprising a lecithinized superoxide dismutase.

## Description

### Technical Field

The present invention relates to a pharmaceutical composition for treating or preventing a disorder associated with administration of an anticancer agent.

### Background Art

With development of many anticancer agents, the effect of drug therapies on cancer has been dramatically improved. However, adverse drug reactions caused by anticancer agents have also become evident. In particular, various disorders associated with administration of anticancer agents, including neurological disorders, auditory disorders, disorders of organs such as liver and kidneys, and myelosuppression, have been long known. Occurrence or aggravation of these disorders significantly lowers the QOL of patients, thereby affecting daily activities.

Among these disorders, as drugs for neurological disorders, a blood glucose modulator (Patent Literature 1), a motor neurotrophic factor peptide analogue (Patent Literature 2), a glutamate dehydrogenase gene (Patent Literature 3), a CYP2J2 antagonist (Patent Literature 4), a CXCR2 antagonist (Patent Literature 5), a selective organic cation transporter (Patent Literature 6), neuplastin (Patent Literature 7), a GLP-1 analogue derivative (Patent Literature 8), and calmangafodipir (Patent Literature 9) have been reported to be effective.

### Citation List

### Patent Literature

Patent Literature 1: JP-A-2003-532691
Patent Literature 2: JP-A-2006-516287
Patent Literature 3: JP-A-2008-518599
Patent Literature 4: JP-A-2017-524696
Patent Literature 5: JP-A-2017-527542
Patent Literature 6: JP-A-2018-521133
Patent Literature 7: JP-B-3738008
Patent Literature 8: JP-B-6231386
Patent Literature 9: JP-B-6131271
Patent Literature 10: JP-A-2006-169128
Patent Literature 11: JP-A-2008-75706
Patent Literature 12: JP-A-2010-215527
Patent Literature 13: JP-A-2017-73255
Patent Literature 14: JP-A-2001-64199
Patent Literature 15: JP-A-6-199895
Patent Literature 16: WO-A-2006-040980

### Summary of the Invention

### Technical Problem

However, no treating or relieving drug has been shown to have a sufficient effect, or development of such drugs was discontinued because of issues such as adverse reactions. As such, there is an urgent unmet need for development of a novel treating or preventing drug.

Therefore, the present invention aims to provide a pharmaceutical composition for treating or preventing a disorder associated with administration of an anticancer agent.

### Solution to Problem

Accordingly, the present inventors focused on a lecithinized superoxide dismutase (PC-SOD) and examined a pharmacological action thereof from various points of view. As a result, the present inventors found that PC-SOD is effective for neurological disorders attributed to administration of an anticancer agent, such as chemotherapy-induced peripheral neuropathy (CIPN), neuropathic pain, allodynia, and thermal sensation disorder and also found that PC-SOD is also effective for liver disorders, drug-induced deafness, kidney disorders, and myelosuppression attributed to administration of an anticancer agent and is highly safe, and thus accomplished the present invention.

Specifically, the present invention provides the following [1] to [29]:
[1] A pharmaceutical composition comprising a lecithinized superoxide dismutase, for treating or preventing a disorder associated with administration of an anticancer agent.
[2] The pharmaceutical composition according to [1], wherein the disorder associated with administration of an anticancer agent is a disorder selected from the group consisting of a neurological disorder, a liver disorder, drug-induced deafness, a kidney disorder, myelosuppression, and a combination of one or more thereof.
[3] The pharmaceutical composition according to [2], wherein the neurological disorder is a peripheral neurological disorder, neuropathy, neuropathic pain, allodynia, hyperalgesia or hypoalgesia, or thermal sensation disorder.
[4] The pharmaceutical composition according to [2], wherein the neurological disorder is a neurological disorder resulting from decreased nerve fiber density, reduced or retracted neurites, or damaged dorsal root ganglion (DRG) neurons.
[5] The pharmaceutical composition according to [1], wherein the disorder associated with administration of an anticancer agent is chemotherapy-induced peripheral neuropathy (CIPN).
[6] The pharmaceutical composition according to [1] or [2], wherein the disorder associated with administration of an anticancer agent is a liver disorder, and the anticancer agent is a platinum-based drug.
[7] The pharmaceutical composition according to [1] or [2], wherein the disorder associated with administration of an anticancer agent is drug-induced deafness, and the anticancer agent is platinum-based drug.
[8] The pharmaceutical composition according to [1] or [2], wherein the disorder associated with administration of an anticancer agent is a kidney disorder, and the anticancer agent is platinum-based drug.
[9] The pharmaceutical composition according to [8], wherein the kidney disorder is cisplatin-induced nephropathy, and the anticancer agent is cisplatin.
[10] The pharmaceutical composition according to [8] or [9], wherein the kidney disorder is nephropathy with oliguria.
[11] The pharmaceutical composition according to any one of [8] to [10], wherein the kidney disorder is in a transitional phase from an acute kidney disorder to a chronic kidney disorder.
[12] The pharmaceutical composition according to [1] or [2], wherein the disorder associated with administration of an anticancer agent is myelosuppression, and the anticancer agent is a platinum-based drug or a metabolism antagonist.
[13] The pharmaceutical composition according to any one of [1] to [5], wherein the anticancer agent is at least one selected from the group consisting of a platinum-based drug, a metabolism antagonist, a taxane drug, a vinca alkaloid drug, and a proteasome inhibitor.
[14] The pharmaceutical composition according to [13], wherein the anticancer agent is a platinum-based drug.
[15] The pharmaceutical composition according to [13] or [14], wherein the anticancer agent is oxaliplatin or cisplatin.
[16] The pharmaceutical composition according to any one of [1] to [15], wherein the anticancer agent is administered in FOLFOX therapy or XELOX therapy (CapeOX therapy).
[17] The pharmaceutical composition according to any one of [1] to [16], wherein the anticancer agent is administered to a cancer patient suffering from one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, trophoblastic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer.
[18] The pharmaceutical composition according to any one of [1] to [16], wherein the anticancer agent is administered to a patient who has a medical history of one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, chorionic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer and from whom the cancer or cancers were removed.
[19] The pharmaceutical composition according to any one of [1] to [18], which is administered in combination with an anticancer agent.
[20] The pharmaceutical composition according to any one of [1] to [19], wherein the lecithinized superoxide dismutase is a lyophilized formulation.
[21] The pharmaceutical composition according to any one of [1] to [20], comprising a lecithinized superoxide dismutase,
   the lecithinized superoxide dismutase comprising a lecithinized superoxide dismutase in which at least one amino group in a human-derived superoxide dismutase subunit coordinated with copper and/or zinc and having a mercapto group of cysteine at position 111 replaced by a hydroxyethylthio group is modified with a lecithin residue of the following formula (I): 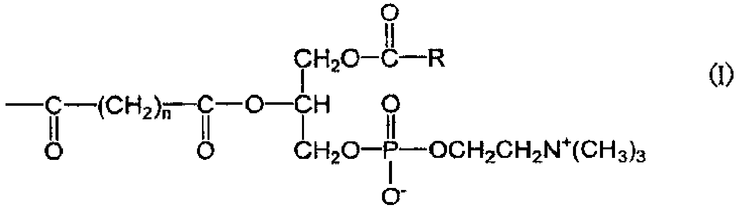
      [wherein R represents an alkyl group with 8 to 30 carbon atoms, and n is an integer of from 2 to 10]
      directly or via a linker, and
   the lecithinized superoxide dismutase comprises a lecithinized superoxide dismutase (A), as a principal component, constituted by lecithinized superoxide dismutase subunits each of which independently has m number of amino groups [m is an integer of from 1 to 4, and the mean value of m in lecithinized superoxide dismutase subunits is from 1.5 to 2.4] substituted with the lecithin residue, wherein
   the lecithinized superoxide dismutase (A) is constituted by the following lecithinized superoxide dismutase subunits:
      from 25 to 40 mol% lecithinized superoxide dismutase subunit (a1) with m = 1,
      from 35 to 50 mol% lecithinized superoxide dismutase subunit (a2) with m = 2,
      from 10 to 20 mol% lecithinized superoxide dismutase subunit (a3) with m = 3, and
      from 5 to 15 mol% lecithinized superoxide dismutase subunit (a4) with m = 4.
[22] The pharmaceutical composition according to any one of [1] to [21], wherein PC bind to SOD with the following binding rates in the lecithinized superoxide dismutase:
   (1) the binding rate of the PC at the binding sites of K3, N terminus, and T2 is from 1% to 10% as the sum of these amino acid residues;
   (2) the binding rate of the PC at the binding site of K9 is from 10% to 30%;
   (3) the binding rate of the PC at the binding site of K23 is from 50% to 75%;
   (4) the binding rate of the PC at the binding site of K36 is from 30% to 55%;
   (5) the binding rate of the PC at the binding site of K70 or K75 is from 10% to 80% as the sum of these amino acid residues;
   (6) the binding rate of the PC at the binding site of K122 or K128 is from 10% to 45% as the sum of these amino acid residues;
   (7) the binding rate of the PC at binding site of K136 is from 10% to 60%; and
   as the sum of the (1) to (7), on average 1 to 8 PC molecules are bound in the PC-SOD.
[23] The pharmaceutical composition according to any one of [1] to [22], wherein a superoxide dismutase in the lecithinized superoxide dismutase has an amino acid sequence set forth in SEQ ID NO: 1.
[24] Use of a lecithinized superoxide dismutase for manufacturing a pharmaceutical composition for treating or preventing a disorder associated with administration of an anticancer agent.
[25] The use according to [24], wherein the disorder associated with administration of an anticancer agent is a disorder selected from the group consisting of a neurological disorder, a liver disorder, drug-induced deafness, a kidney disorder, myelosuppression, and a combination of one or more thereof.
[26] A lecithinized superoxide dismutase for use in treating or preventing a disorder associated with administration of an anticancer agent.
[27] The lecithinized superoxide dismutase according to [26], wherein the disorder associated with administration of an anticancer agent is a disorder selected from the group consisting of a neurological disorder, a liver disorder, drug-induced deafness, a kidney disorder, myelosuppression, and a combination of one or more thereof.
[28] A method for treating or preventing a disorder associated with administration of an anticancer agent, comprising administration of a lecithinized superoxide dismutase.
[29] The method according to [28], wherein the disorder associated with administration of an anticancer agent is a disorder selected from the group consisting of a neurological disorder, a liver disorder, drug-induced deafness, a kidney disorder, myelosuppression, and a combination of one or more thereof.

### Advantageous Effects of Invention

The PC-SOD used in the present invention is useful for use in preventing or treating various disorders associated with administration of an anticancer agent, specifically, a neurological disorder, a liver disorder, drug-induced deafness, a kidney disorder, myelosuppression, or a combination of one or more thereof, particularly preferably CIPN and the like, and is highly safe.

### Brief Description of Drawings

Fig. 1 shows the concentration-dependent results of prophylactic administration of PC-SOD (OXA + PC-SOD) to oxaliplatin (OXA)-induced allodynia model rats.
Fig. 2 shows a prophylactic effect of PC-SOD (OXA + PC-SOD) on cold allodynia attributed to oxaliplatin (OXA) .
Fig. 3 shows the results of administration of PC-SOD (OXA + PC-SOD) against decreased density of intraepidermal nerve fiber caused by oxaliplatin (OXA).
Fig. 4 shows the results of administration of PC-SOD (OXA +PC-SOD) against pathological damage to dorsal root ganglion (DRG) neurons caused by oxaliplatin (OXA).
Fig. 5 shows the results of administration of PC-SOD (OXA +PC-SOD) against pathological damage to the liver caused by oxaliplatin (OXA).
Fig. 6 shows an influence of administration of PC-SOD (PC-SOD) on the inhibitory effect of oxaliplatin (OXA) on proliferation of large intestine cancer cells.
Fig. 7-1 shows an action of administration of PC-SOD (PC-SOD) on neurite retraction caused by addition of a platinum-based drug (cisplatin (+ NGF + CP) or oxaliplatin (+ NGF + OXA)).
Fig. 7-2 shows an action of administration of PC-SOD (PC-SOD) on neurite retraction caused by addition of a platinum-based drug (carboplatin).
Fig. 7-3 shows an action of administration of PC-SOD (PC-SOD) on neurite retraction caused by addition of paclitaxel (+ NGF + PTX).
Fig. 8 shows an action of administration of PC-SOD (PC-SOD) and an action of administration of mangafodipir (Mangafodipir) on neurite retraction caused by addition of oxaliplatin (+ NGF + OXA).
Fig. 9 shows nerve cell toxicity of PC-SOD (PC-SOD) and that of mangafodipir in the presence and absence of oxaliplatin.
Fig. 10-1 shows an effect of PC-SOD (PC-SOD) on a kidney disorder caused by cisplatin (CIS) (a photograph of the kidneys).
Fig. 10-2 shows an effect of PC-SOD (PC-SOD) on a kidney disorder caused by cisplatin (CIS) (changes in kidney weight).
Fig. 11-1 shows an effect of PC-SOD (PC-SOD) on a kidney disorder (pathological damage to the kidneys) caused by cisplatin (CIS).
Fig. 11-2 shows an effect of PC-SOD (PC-SOD) on a kidney disorder (renal fibrosis) caused by cisplatin (CIS) (picrosirius red staining).
Fig. 11-3 shows an effect of PC-SOD (PC-SOD) on a kidney disorder (renal fibrosis) caused by cisplatin (CIS) (α-SMA staining).
Fig. 12-1 shows cytotoxicity of anticancer agents (gemcitabine, oxaliplatin, cisplatin, and carboplatin) against RAW cells.
Fig. 12-2 shows an effect of PC-SOD on cytotoxicity of anticancer agents (gemcitabine, oxaliplatin, cisplatin, and carboplatin) against RAW cells.
Fig. 13-1 shows active oxygen production action in RAW cells induced by anticancer agents (gemcitabine, oxaliplatin, cisplatin, and carboplatin).
Fig. 13-2 shows an effect of PC-SOD on active oxygen production in RAW cells induced by anticancer agents (gemcitabine, oxaliplatin, cisplatin, and carboplatin).
Fig. 14 shows an effect of PC-SOD (PC-SOD) on myelosuppression (changes in blood cells in peripheral blood) by cisplatin (CIS).
Fig. 15 shows PC modification site and percent PC modification of PC-SOD obtained by enzyme digestion and LC/MS analysis of PC-SOD.

### [Description of Embodiments]

An active ingredient of the pharmaceutical composition for treating or preventing a disorder associated with administration of an anticancer agent of the present invention is a lecithinized superoxide dismutase (PC-SOD).

In the present specification, the terms "lecithinized superoxide dismutase" and "PC-SOD" are synonymous and can be used interchangeably.

In the present specification, the terms "superoxide dismutase" and "SOD" are synonymous and can be used interchangeably.

In the present specification, the terms "phosphatidylcholine," "PC," and "lecithin" are synonymous, can be used interchangeably, and can be used for both an aggregate and individual molecules.

In the present specification, the term "PC-SOD" means an aggregate of PC-SOD molecules. Here, the number of PC molecules binding to an individual PC-SOD molecule constituting "PC-SOD" and binding sites thereof may be the same or different between PC-SOD molecules. Therefore, the number of PC molecules binding to "PC-SOD" can be represented by the average number of binding PC molecules. In "PC-SOD", the PC binding site can be identified by an amino acid residue in the SOD, and the binding rate of the PC can be represented by the percent of PC molecules binding to amino acid residues in an SOD.

In the present specification, the term "PC-SOD subunit" means an aggregate of PC-SOD molecule subunits.

In the present specification, the term "SOD" means an aggregate of SOD molecules.

In the present specification, the term "SOD subunit" means an aggregate of SOD molecule subunits.

In the present specification, the term "PC-SOD molecule" means a dimeric molecule consisting of PC-SOD molecule subunits, and the term "PC-SOD molecule subunit" means a monomer constituting a dimeric PC-SOD molecule. Here, the number and the binding site of PC molecules binding a PC-SOD molecule subunit constituting each PC-SOD molecule dimer may be the same or different between PC-SOD molecule subunits constituting the dimer.

In the present specification, the term "SOD molecule" means a molecule obtained by dimerizing SOD molecule subunits, and the term "SOD molecule subunit" means a monomer constituting a dimeric SOD molecule.

PC-SOD is a molecule obtained by modifying SOD with lecithin. The applicant of the present application reported so far that PC-SOD is effective for burn (Patent Literature 10), interstitial pneumonia (Patent Literature 11), chronic obstructive pulmonary disease syndrome (Patent Literature 12), acute respiratory distress syndrome (Patent Literature 13), and the like. However, the action of PC-SOD on a disorder associated with administration of an anticancer agent is unknown at all.

PC-SOD is a molecule obtained by modifying SOD with lecithin and is preferably a molecule obtained by modifying one or more amino acid residues of SOD, preferably an amino acid residue containing an amino group or an N-terminal amino acid residue, more preferably a lysine residue with phosphatidylcholine (PC) directly or via a linker.

As SOD in the PC-SOD, a human SOD (SEQ ID NO: 1) is preferred, and a human SOD having copper and zinc at the active center (human Cu/Zn SOD) is particularly preferred. Examples of this SOD include a natural human Cu/Zn SOD manufactured from human tissue, a human Cu/Zn SOD manufactured by using a genetic engineering technique, a recombinant human Cu/Zn SOD having an amino acid sequence substantially identical to that of a natural human Cu/Zn SOD, and an SOD obtained by chemically modifying or altering some amino acids in the amino acid sequences of these human Cu/Zn SOD molecules, and any one of these human Cu/Zn SOD molecules may be used. The SOD in PC-SOD is preferably a human SOD 1, more preferably a human SOD 1 in which a mercapto group of cysteine at position 111 is protected, further more preferably a human SOD 1 in which a mercapto group of cysteine at position 111 is replaced by a hydroxyethylthio group.

In PC-SOD, PC binds directly or via a linker to one or more free amino groups or hydroxy groups (preferably amino groups) among amino acid residues constituting SOD. Examples of an amino acid residue to which PC binds include Ala at the N terminus of an SOD molecule and Lys, Gln, Asn, Arg, Ser, Thr, and Tyr, preferably Ala at the N terminus and Lys and Thr, more preferably Ala at the N terminus and Lys, further more preferably Lys.

Preferably 1 to 12 PC molecules, more preferably 1 to 8 PC molecules, further more preferably 1 to 4 PC molecules, even more preferably 2 to 3 PC molecules, further preferably 2 PC molecules are independently bound to each SOD molecule subunit.

Therefore, preferably 2 to 24 PC molecules, more preferably 2 to 16 PC molecules, further more preferably 2 to 8 PC molecules, even more preferably 4 to 6 PC molecules, further more preferably 4 PC molecules are bound to a PC-SOD molecule.

For PC-SOD, the number of PC molecules binding to a SOD molecule can be expressed with the average number of PC molecules binding to an aggregate of SOD molecules. Therefore, in PC-SOD, 2 to 24 PC molecules, preferably 2 to 16 PC molecules, more preferably 2 to 8 PC molecules, further more preferably 4 to 6 PC molecules, most preferably 4 PC molecules are bound to an SOD molecule on average.

PC may bind to an amino acid residue constituting SOD directly, but preferably via a linker.

As a linker, dicarboxylic acid is preferred, - C(=O) (CH₂)ₙC(=O)- is particularly preferred (here, n is an integer of 2 or greater, preferably from 2 to 10, more preferably from 2 to 6, further more preferably from 2 to 4, particularly preferably 3). It should be noted that this linker links a hydroxy group of a glyceryl group in PC and an amino group or a hydroxy group (preferably amino group) of an amino acid residue.

A fatty acid residue in PC has preferably 8 to 31 carbon atoms, more preferably 10 to 28 carbon atoms, further more preferably 14 to 22 carbon atoms, particularly preferably 16 carbon atoms (derived from palmitic acid).

PC-SOD can be manufactured by, for example, the methods described in Patent Literatures 14, 15, and 16, preferably the method described in Patent Literature 16.

A particularly preferred example of PC-SOD is a lecithinized superoxide dismutase,
the lecithinized superoxide dismutase comprising a lecithinized superoxide dismutase in which at least one amino group in a human-derived superoxide dismutase subunit coordinated with copper and/or zinc and having a mercapto group in cysteine at position 111 replaced by a hydroxyethylthio group is modified with a lecithin residue of the following formula (I): 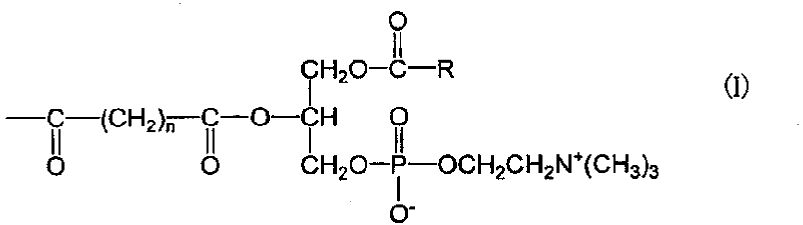
   [wherein R represents an alkyl group with 8 to 30 carbon atoms, and n is an integer of from 2 to 10]
   directly or via a linker, wherein
the lecithinized superoxide dismutase comprises a lecithinized superoxide dismutase (A), as a main component, constituted by lecithinized superoxide dismutase subunits each of which independently has m of amino groups [m is an integer of from 1 to 4, and the mean value of m in lecithinized superoxide dismutase subunits is from 1.5 to 2.4] substituted with the lecithin residue, wherein
the lecithinized superoxide dismutase (A) is constituted by the following lecithinized superoxide dismutase subunits:
   from 25 to 40 mol% lecithinized superoxide dismutase subunit (a1) with m = 1,
   from 35 to 50 mol% lecithinized superoxide dismutase subunit (a2) with m = 2,
   from 10 to 20 mol% lecithinized superoxide dismutase subunit (a3) with m = 3, and
   from 5 to 15 mol% lecithinized superoxide dismutase subunit (a4) with m = 4.

In the PC-SOD of the present invention, the binding sites and the binding rates of PC binding to SOD are preferably as described below. It should be noted that the number after a one-letter amino acid code represents the number of residues in an SOD molecule from the N terminus.
(1) When the binding site is K3, the N terminus, or T2, the binding rate of the PC is preferably from 1% to 10%, more preferably from 2% to 8%, further more preferably 5% as the sum of these amino acid residues.
(2) When the binding site is K9, the binding rate of the PC is preferably from 10% to 30%, more preferably from 15% to 26%, further more preferably 23%.
(3) When the binding site is K23, the binding rate of the PC is preferably from 50% to 75%, more preferably from 55% to 70%, further more preferably 63%.
(4) When the binding site is K36, the binding rate of the PC is preferably from 30% to 55%, more preferably from 35% to 50%, further more preferably 43%.
(5) When the binding site is K70 or K75, the binding rate of the PC is preferably from 10% to 80%, more preferably from 30% to 65%, further more preferably 53% as the sum of these amino acid residues.
(6) When the binding site is K122 or K128, the binding rate of the PC is preferably from 10% to 45%, more preferably from 20% to 35%, further more preferably 27% as the sum of these amino acid residues.
(7) When the binding site is K136, the binding rate of the PC is preferably from 10% to 60%, more preferably from 20% to 45%, further more preferably 33%.

In a preferred embodiment, as the sum of the above-mentioned (1) to (7), 1 to 8 PC molecules, preferably 2 to 6 PC molecules, more preferably 3 to 5 PC molecules, most preferably 4 PC molecules bind to PC-SOD on average.

As described in the examples described later, the PC-SOD has a therapeutic or prophylactic effect on a disorder associated with administration of an anticancer agent. Here, examples of the disorder associated with administration of an anticancer agent include a neurological disorder, a liver disorder, drug-induced deafness, a kidney disorder, myelosuppression, and an arbitrary combination of one or more thereof.

Since patients who have received an anticancer agent, in particular, a platinum-based drug, often develop a neurological disorder, that the present invention is effective for a neurological disorder as a disorder associated with administration of an anticancer agent suggests that the present invention is beneficial for improvement of the QOL of patients receiving an anticancer agent.

Since patients who have received an anticancer agent often develop a liver disorder attributed to the anticancer agent, that the present invention is effective for a liver disorder suggests that the present invention is beneficial for improvement of the QOL of patients receiving an anticancer agent.

Since patients who have received an anticancer agent, in particular, a platinum-based drug, often develop drug-induced deafness, that the present invention is effective for drug-induced deafness as a disorder associated with administration of an anticancer agent suggests that the present invention is beneficial for improvement of the QOL of patients receiving an anticancer agent.

Since patients who have received an anticancer agent, in particular, a platinum-based drug, more preferably cisplatin, often develop a kidney disorder, that the present invention is effective for a kidney disorder as a disorder associated with administration of an anticancer agent suggests that the present invention is beneficial for improvement of the QOL of patients receiving an anticancer agent.

Since patients who have received an anticancer agent, in particular, a platinum-based drug or a metabolism antagonist, more preferably cisplatin, oxaliplatin, carboplatin, gemcitabine, or the like, often develop myelosuppression, that the present invention is effective for myelosuppression as a disorder associated with administration of an anticancer agent suggests that the present invention is beneficial for improvement of the QOL of patients receiving an anticancer agent.

The pharmaceutical composition of the present invention is useful for a peripheral neurological disorder, neuropathy, neuropathic pain, allodynia, hyperalgesia or hypoalgesia, or thermal sensation disorder among neurological disorders associated with administration of an anticancer agent and is particularly useful for CIPN. The term allodynia used herein is synonymous with *itsusho* in Japanese.

Specific examples of symptoms of the neuropathic pain or allodynia include one or more selected from the group consisting of numbness in the extremities, pain in the extremities, decreased deep tendon reflex, muscle weakness, and motor function disorder.

Examples of symptoms of the thermal sensation disorder include one or more selected from the group consisting of cold hypersensitivity, cold hypoesthesia, heat hypersensitivity, and heat hypoesthesia.

These symptoms are disorders associated with administration of an anticancer agent, which are particularly recognized as chemotherapy-induced peripheral neuropathy (CIPN) and decrease the QOL of cancer patients. Therefore, it is highly important in performing cancer chemotherapy that the pharmaceutical composition of the present invention can treat or prevent these symptoms.

The pharmaceutical composition of the present invention exhibits a therapeutic or prophylactic effect against decreases in density of intraepidermal nerve fiber resulting from administration of an anticancer agent and a prophylactic effect on pathological damage to dorsal root ganglion (DRG) neurons attributed to administration of an anticancer agent. Further, the pharmaceutical composition of the present invention has a protective action on neurites from disorders resulting from administration of an anticancer agent.

Therefore, the pharmaceutical composition of the present invention has a therapeutic or prophylactic effect on a neurological disorder resulting from decreased nerve fiber density, a neurological disorder resulting from reduced or retracted neurites, and a neurological disorder resulting from damaged dorsal root ganglion (DRG) neurons.

The pharmaceutical composition of the present invention has a therapeutic or prophylactic effect on a liver disorder associated with administration of an anticancer agent.

The pharmaceutical composition of the present invention has a therapeutic or prophylactic effect on drug-induced deafness associated with administration of an anticancer agent, in particular, administration of a platinum-based drug, more preferably administration of oxaliplatin, carboplatin, or cisplatin.

The pharmaceutical composition of the present invention has a therapeutic or prophylactic effect on a kidney disorder associated with administration of an anticancer agent, in particular, administration of a platinum-based drug, more preferably administration of cisplatin. The kidney disorder associated with administration of an anticancer agent is preferably nephropathy with oliguria or cisplatin-induced nephropathy. The kidney disorder associated with administration of an anticancer agent is preferably in a transitional phase from an acute kidney disorder to a chronic kidney disorder.

The pharmaceutical composition of the present invention has a therapeutic or prophylactic effect on myelosuppression associated with administration of an anticancer agent, in particular, administration of a platinum-based drug or administration of a metabolism antagonist, more preferably administration of oxaliplatin, carboplatin, cisplatin, gemcitabine, or the like.

Furthermore, the pharmaceutical composition of the present invention is also characterized by not affecting the anti-tumor effect of an anticancer agent, being less cytotoxic than calmangafodipir described in Patent Literature 9, and having a high neuroprotective action.

As described above, the pharmaceutical composition of the present invention has a therapeutic or prophylactic effect on a disorder associated with administration of an anticancer agent. The anticancer agent is preferably a chemotherapeutic agent, preferably at least one selected from the group consisting of a platinum-based drug, a metabolism antagonist, a taxane drug, a vinca alkaloid drug, and a proteasome inhibitor, particularly preferably a platinum-based drug.

Specific examples of the platinum-based drug include cisplatin, carboplatin, and oxaliplatin.

Specific examples of the taxane drug include paclitaxel and docetaxel.

Specific examples of the vinca alkaloid drug include vincristine or vinorelbine.

Specific examples of the proteasome inhibitor include bortezomib and carfilzomib.

Specific examples of the metabolism antagonist include gemcitabine, cytarabine, carmofur, tegafur, 5-fluorouracil, methotrexate, and capecitabine.

Specific examples of an anticancer agent include preferably at least one selected from the group consisting of doxorubicin, epirubicin, oxaliplatin, carboplatin, cisplatin, gemcitabine, 5-fluorouracil, capecitabine, docetaxel, paclitaxel, vincristine, vinblastine, vinorelbine, bortezomib, and thalidomide, particularly preferably oxaliplatin.

Preferred examples of a combination of a disorder associated with administration of an anticancer agent and an anticancer agent in the present invention include the following:
(1) When a disorder associated with administration of an anticancer agent is a neurological disorder, the anticancer agent is preferably a platinum-based drug or a taxane drug, and the anticancer agent is more preferably oxaliplatin, cisplatin, or paclitaxel. In particular, when a disorder associated with administration of an anticancer agent is CIPN, the anticancer agent is preferably a platinum-based drug, more preferably oxaliplatin.
(2) When a disorder associated with administration of an anticancer agent is a liver disorder, the anticancer agent is preferably a platinum-based drug, particularly preferably oxaliplatin.
(3) When a disorder associated with administration of an anticancer agent is drug-induced deafness, the anticancer agent is preferably a platinum-based drug, particularly preferably cisplatin.
(4) When a disorder associated with administration of an anticancer agent is a kidney disorder, the anticancer agent is preferably a platinum-based drug. In particular, when a disorder associated with administration of an anticancer agent is cisplatin-induced nephropathy, the anticancer agent is preferably cisplatin.
(5) When a disorder associated with administration of an anticancer agent is myelosuppression, the anticancer agent is preferably a platinum-based drug or a metabolism antagonist, particularly preferably cisplatin, carboplatin, oxaliplatin, or gemcitabine.

Additionally, the pharmaceutical composition of the present invention is preferably administered in cancer chemotherapy, for example, FOLFOX therapy or XELOX therapy (CapeOX therapy), more preferably mFOLFOX therapy.

FOLFOX is a cancer chemotherapy in which folinic acid, fluorouracil, and oxaliplatin are used in combination. FOLFOX has variations of modified methods depending on the drug dose differences, including FOLFOX1 to FOLFOX7, and mFOLFOX6, and FOLFOX4, FOLFOX6, and mFOLFOX6 are preferred. mFOLFOX6 is particularly preferred in Japan.

XELOX therapy (CapeOX therapy) is a cancer chemotherapy in which Xeloda and oxaliplatin (capecitabine and oxaliplatin) are used in combination.

The pharmaceutical composition of the present invention is preferably administered to a cancer patient suffering from one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, chorionic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer, more preferably a cancer patient suffering from one or more cancers selected from the group consisting of stomach cancer, pancreas cancer, small intestine cancer, colon cancer, rectal cancer, and large intestine cancer, further more preferably a cancer patient suffering from one or more cancers selected from the group consisting of colon cancer, rectal cancer, and large intestine cancer.

In the present invention, an anticancer agent can also be administered as a postoperative adjuvant chemotherapy to a patient from whom cancer was removed or to a patient to prevent recurrence. Specifically, an anticancer agent is administered preferably to a patient who has a medical history of one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, chorionic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer and from whom the cancer is removed, more preferably a patient who has a medical history of one or more cancers selected from the group consisting of stomach cancer, pancreas cancer, small intestine cancer, colon cancer, rectal cancer, and large intestine cancer and from whom the cancer or cancers were removed, further more preferably a patient who has a medical history of one or more cancers selected from the group consisting of colon cancer, rectal cancer, and large intestine cancer and from whom the cancer or cancers were removed.

Since the pharmaceutical composition of the present invention has a therapeutic or prophylactic effect on not only a neurological disorder associated with administration of an anticancer agent, but also a liver disorder, drug-induced deafness, and/or a kidney disorder, it can also be administered to a patient who concurrently has an arbitrary combination of one or more of a neurological disorder, a liver disorder, drug-induced deafness, a kidney disorder, and myelosuppression.

Since the pharmaceutical composition of the present invention has a therapeutic or prophylactic effect on a neurological disorder, a liver disorder, drug-induced deafness, a kidney disorder, myelosuppression, or a combination of one or more thereof associated with administration of an anticancer agent, the pharmaceutical composition of the present invention is preferably administered in combination with an anticancer agent.

The pharmaceutical composition of the present invention is not limited as long as it comprises PC-SOD, and a pharmaceutical composition is preferably formulated in various dosage forms by mixing pharmacologically acceptable carriers with PC-SOD.

Such a pharmaceutical composition is administered by injection, preferably intravenous injection, local injection, or drip infusion.

For injection, water, a dissolving aid, a xylitol aqueous solution (preferably 5 w/w%, 10 w/w%, or 20 w/w%, more preferably 5 w/w%), or the like is used. An injection is preferably an intravenous injection, a local injection, a subcutaneous injection, and an intramuscular injection.

Examples of dosage forms of the pharmaceutical composition of the present invention include forms of a lyophilized formulation and a powder filler, and a lyophilized formulation is preferred. To prepare a lyophilized formulation, a stabilizer such as sucrose is preferably mixed. The formulation is prepared by lyophilizing an aqueous solution containing PC-SOD and sucrose by a method known in this technical field. The weight ratio of PC-SOD and sucrose is preferably from 1:1 to 1:5, more preferably from 1:1 to 1:2. For 40 mg of PC-SOD, from 40 to 200 mg of sucrose is preferred, from 40 to 80 mg is more preferred, and 67 mg is further more preferred.

The content of PC-SOD in the pharmaceutical composition of the present invention is not particularly limited, but is usually from 1 to 100 mass%, preferably from 10 to 80 mass%, more preferably from 25 to 50 mass% to the total mass of a composition.

Doses of the pharmaceutical composition of the present invention vary depending on symptoms, age, and the like of a patient to whom an anticancer agent is administered, and from 20 to 160 mg per day of the PC-SOD for adults is preferred, from 20 to 80 mg is more preferred, and from 40 to 80 mg is further more preferred. The dose can be divided into one to four times per week, one to four times per two weeks, or one to four times per three weeks, and each dosing cycle can be repeated from once to 20 times. Preferably, the pharmaceutical composition of the present invention can be administered in accordance with the FOLFOX or XELOX dosing regimen. With the FOLFOX dosing regimen, the pharmaceutical composition of the present invention is preferably administered once every two weeks which is repeated 12 cycles. With the XELOX dosing regimen, the pharmaceutical composition of the present invention is preferably administered once every three weeks which is repeated eight cycles.

Another preferred embodiment of the present invention is a PC-SOD for preventing or treating a disorder associated with administration of an anticancer agent.

Another preferred embodiment of the present invention is use of a PC-SOD to manufacture a pharmaceutical composition for preventing or treating a disorder associated with administration of an anticancer agent.

Another preferred embodiment of the present invention is a method for preventing or treating a disorder associated with administration of an anticancer agent, comprising administration of a PC-SOD.

### Examples

The present invention is described in detail below with reference to examples, but the scope of the present invention is not limited to these examples.

The PC-SOD used in these examples was manufactured in accordance with the method described in Patent Literature 16.

### [Test Example 1] Action of oxaliplatin on rat allodynia "Summary"

To determine the effect of PC-SOD on allodynia attributed to an anticancer agent, the action of PC-SOD on allodynia was investigated by using a mechanical stimulus resulting from administration of an anticancer agent oxaliplatin to rats. PC-SOD was administered into the caudal vein of rats as a test drug, and the following tests were performed.

### "Method"

### (1) Preparation of oxaliplatin-induced allodynia model rats

Six to eight-week-old male SD rats (from 200 to 400 g) were used, and oxaliplatin (hereinafter may be abbreviated as OXA) was administered into the peritoneal cavity of rats at a dose of 4 mg/kg. A total of eight doses were given with a regimen of twice weekly for four weeks (Days 1, 2, 7, 8, 13, 14, 19, and 20). A solvent for OXA, i.e., a 5% glucose solution, was similarly administered to a control group. Elplat I.V. Infusion Solution 100 mg (Yakult Honsha Co., Ltd.) was used as OXA.

### (2) Experiment to assess the prophylactic effect of administration of the test drug

Laboratory animals were divided into five groups: a control group, an OXA administration group, and groups each receiving administration of OXA and PC-SOD 0.1, 0.3, or 1 mg/kg (OXA + PC-SOD administration groups) (n = 8 per group). In an experiment of prevention, PC-SOD was administered into the caudal vein from 5 to 15 minutes after administration of OXA in the OXA + PC-SOD administration group. A total of eight doses were given with a regimen of twice weekly for four weeks (Days 1, 2, 7, 8, 13, 14, 19, and 20). A solvent for PC-SOD was similarly administered to the control group and the OXA administration group.

### (3) Von Frey test

A pressure stimulus was given to the hindlimb of the above-described rats by using a von Frey filament, and a pain sensation test was performed in an unrestrained condition. The strength of the pressure stimulus was set at 50 g.

### (4) Statistical processing

Data were expressed with mean ± standard error (SEM). Statistical processing of the results was performed by Student's t test for comparison of two groups and ANOVA one way test (post-hoc Tukey's test) for comparison of three or more groups, and a statistically significant difference was determined when the hazard ratio was 5% or lower (*, **, and *** in the figures: p < 0.05, p < 0.01, and p < 0.001, respectively).

### "Results and interpretation"

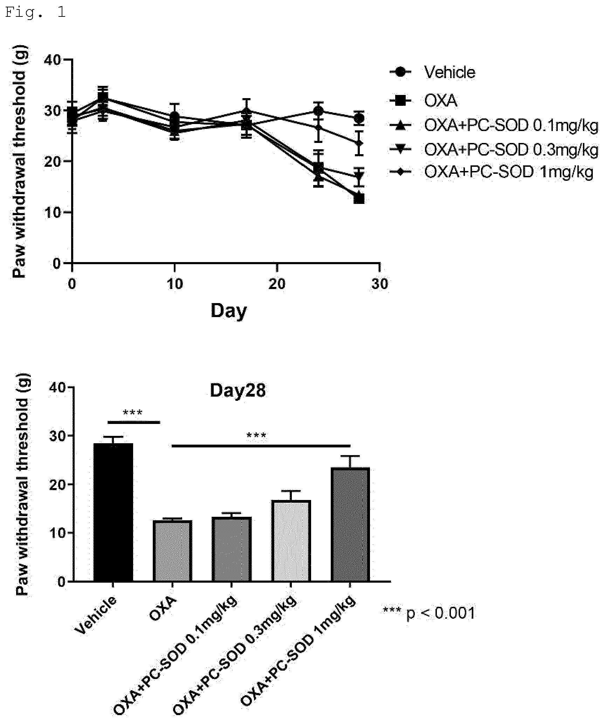

Fig. 1 shows the concentration-dependent results of prophylactic administration of PC-SOD among the results of the above test. In the OXA administration group, the pain sensation threshold significantly decreased as compared with the control group. In contrast, it was shown that the decrease in the pain sensation threshold caused by OXA was suppressed by PC-SOD in a concentration-dependent manner in the OXA + PC-SOD 0.1, 0.3, and 1 mg/kg administration groups. Furthermore, the decrease in the threshold observed in the OXA administration group was significantly suppressed in the PC-SOD 1 mg/kg administration group.

From the above results, it was confirmed that PC-SOD had a prophylactic effect on mechanical allodynia caused by OXA.

### [Test Example 2] Cold stimulation test

### "Summary"

The effect of the present invention against peripheral neuropathic pain attributed to an anticancer agent can be determined by observing allodynia caused by cold stimulation by the method described below.

### "Method"

### (1) Preparation of OXA-induced allodynia model rats

Five-week-old male SD rats (from 200 to 400 g) were used as laboratory animals, and OXA was administered into the peritoneal cavity of rats at a dose of 10 mg/kg. A total of two doses were given (Days 1 and 2). A solvent for OXA, i.e., a 5% glucose solution, was similarly administered to a control group.

### (2) Administration of test drug

Laboratory animals were divided into three groups (n = 8 per group): a control group, an OXA administration group, and a group receiving administration of OXA and PC-SOD 1 mg/kg (OXA + PC-SOD administration group). For the OXA + PC-SOD administration group, PC-SOD was administered into the caudal vein from 15 minutes after administration of OXA as prophylactic administration. A total of two doses were given (Days 1 and 2). A solvent for PC-SOD was similarly administered to the control group and the OXA administration group.

### (3) Acetone test

Rats were placed in a container with a wire net bottom and acclimatized for 30 minutes, and then 0.1 mL of acetone was sprayed onto their hindlimb by using a spray for an organic solvent (No. 3530, Furupla Co., Ltd.) to give a cold stimulus by utilizing a cooling action at the time of vaporization of acetone. Avoidance responses of rats were observed for 40 seconds from the start of spraying and assessed by a scoring method. Specifically, the responses were assessed by scoring with four levels (Scores 0, 1, 2, and 3). Score 0 was to be given if animals were unresponsive as in a resting state. Score 1 was to be given if the hindlimb of animals responded swiftly, but repeated responses, prolonged response time, and the like were not included. Score 2 was to be given if the hindlimb of animals responded swiftly as observed in movement of legs, and repeated responses, prolonged response time, and the like were included. Score 3 was to be given if Score 2 was given and animals started a behavior of licking their hindlimb.

### (4) Statistical processing

Data were expressed with mean ± standard error (SEM). Statistical processing of the results was performed by Student's t test for comparison of two groups and ANOVA one way test (post-hoc Tukey's test) for comparison of three or more groups, and a statistically significant difference was determined if the hazard ratio was 5% or lower (*, **, and *** in the figures: p < 0.05, p < 0.01, and p < 0.001, respectively).

### "Results and interpretation"

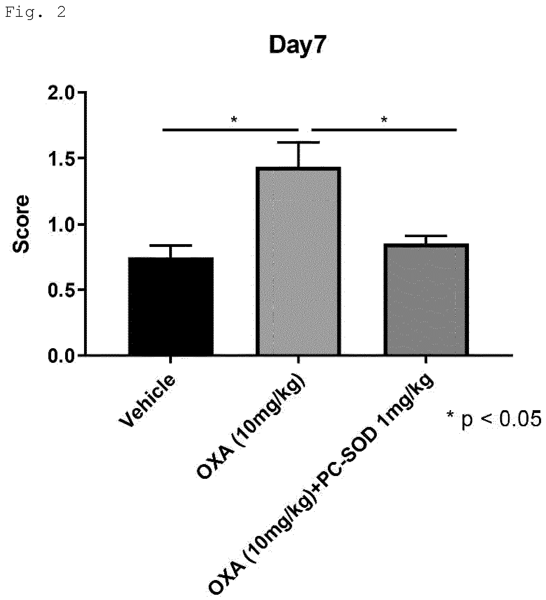

Fig. 2 shows the results of prophylactic administration of PC-SOD against cold allodynia attributed to OXA. In the OXA administration group, the score for cold stimulus increased significantly as compared with the control group. In contrast, the increase in the score observed in the OXA administration group was significantly suppressed in the PC-SOD 1 mg/kg administration group.

From the above results, it was confirmed that PC-SOD had a prophylactic effect on cold allodynia caused by OXA.

### [Test Example 3] Assessment of changes in density of intraepidermal nerve fiber

### "Summary"

The effect of the present invention on pathological damage to peripheral nerves attributed to an anticancer agent can be determined by observing changes in density of intraepidermal nerve fiber caused by oxaliplatin by the method described below.

### "Method"

### (1) Method for assessing intraepidermal nerve density

Rat epidermis tissue was fixed with a 4% paraformaldehyde fixative and washed with PBS 24 hours later, and a frozen block was prepared. A frozen slice having a thickness of 50 µm was prepared from the prepared block by using Cryostat (CM3050S, Leica Biosystems), and a 0.2% Triton-X solution was added as permeabilization treatment to allow the mixture to react for 10 minutes. Nerve fiber was allowed to react with Anti-PGP9.5 antibody (Ultraclone Ltd.) at 4°C overnight, then it was washed with PBST, and allowed to react with a secondary fluorescence antibody (Goat anti-Rabbit IgG Alexa Fluor 488, Invitrogen). After the reaction was done at room temperature for one hour, the nerve fiber was washed, rinsed, followed by dropwise addition of VECTASHIELD with DAPI H-1200 (Vector Laboratories), and enclosed, and two to three images of each sample captured at 200-fold magnification were saved (Scale bar = 100 µm) by using a fluorescence microscope BZ-X800 (Keyence Corporation).

### "Results and interpretation"

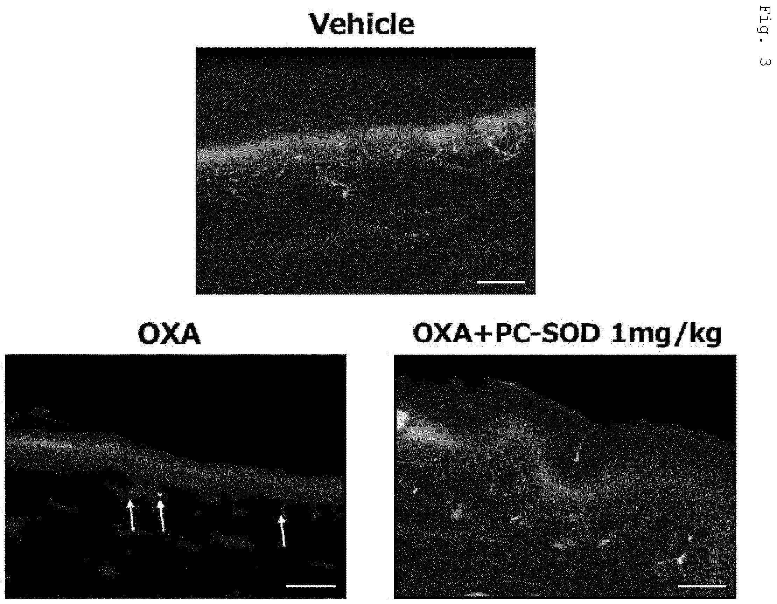

Fig. 3 shows the results of PC-SOD administration against the decrease in density of intraepidermal nerve fiber caused by OXA. In the OXA administration group, the density of intraepidermal nerve fiber decreased as compared with the control group (arrows). In contrast, a suppressive effect on the decrease in density of intraepidermal nerve fiber caused by OXA was observed in the OXA + PC-SOD 1 mg/kg administration group.

From the above results, it was confirmed that PC-SOD had a prophylactic effect on pathological damage to peripheral nerves caused by OXA.

### [Test Example 4] Assessment of pathological damage to DRG "Summary"

The effect of the present invention on pathological damage to DRG neurons attributed to an anticancer agent can be determined by observing morphological changes of DRG caused by oxaliplatin by the method described below.

### "Method"

A tissue of L5 DRG neuron was collected, stored in a 10% neutral buffered formalin solution, fixed, and then washed with PBS. The tissue was subjected to 70% ethanol substitution to prepare a paraffin block, and the block was thinly sliced and subjected to HE staining. The thickness of the thinly sliced sample block was from 2 to 3 µm. By using the prepared pathological sample slide, one to three images of each sample were captured at 200 and 400-fold magnifications by the optical microscope function of All-in-One Fluorescence Microscope (BZ-X800, Keyence Corporation). Multinucleation of the nucleolus of DRG neurons and abnormal morphology of the nucleolus were assessed as indicators of DRG neuron disorder (Scale bar = 50 µm).

### "Results and interpretation"

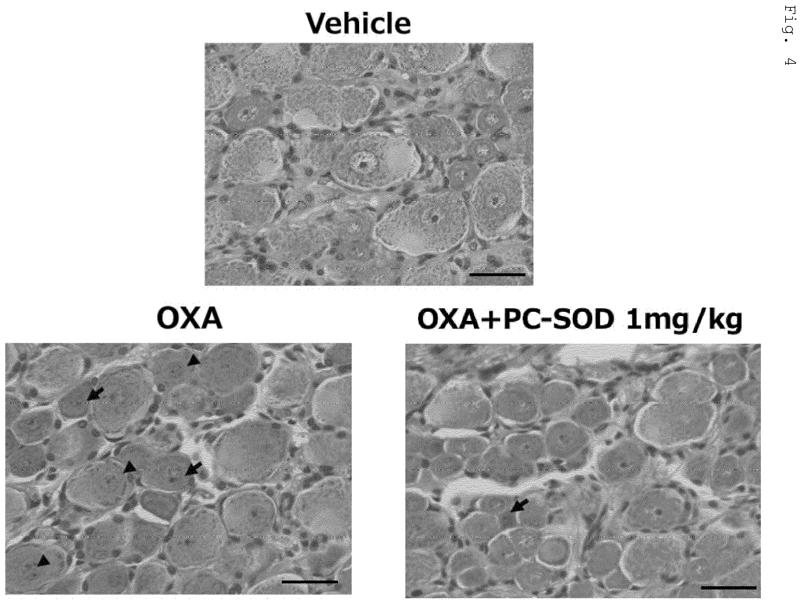

Fig. 4 shows the results of PC-SOD administration against pathological damage to DRG caused by OXA. In the OXA administration group, more pathological damage phenomena were observed as compared with the control group (arrows, multinucleation and abnormal morphology of the nucleoli of DRG neurons). In contrast, a suppressive effect on pathological damage to DRG caused by OXA was shown in the OXA + PC-SOD 1 mg/kg administration group.

From the above results, it was confirmed that PC-SOD had a prophylactic effect on pathological damage to DRG neurons caused by OXA.

### [Test Example 5] Pathological assessment of liver disorder caused by oxaliplatin

### "Summary"

The effect of the present invention on a liver disorder attributed to an anticancer agent and differentiation from mangafodipir can be determined by observing pathological images of the liver disorder caused by oxaliplatin by the method described below.

### "Method"

A liver tissue was collected and fixed with 10% neutral buffered formalin to prepare a paraffin block by using Histos 5 (Milestone) and an embedding center (EG1160, Leica). The sample block was thinly sliced at a thickness of 3 µm by using a microtome (MR2245, Leica) to prepare a slide. The prepared slide was stained by HE staining, enclosed, and observed at 200 and 400-fold magnifications by using the optical microscope function of All-in-One Fluorescence Microscope (BZ-X800, Keyence Corporation). Observation and assessment were performed by observing the whole section. Two to four representative tissue images were randomly taken up, and alteration in the tissue structure, such as inflammation and necrosis, was observed and assessed (Scale bar = 200 µm).

### "Results and interpretation"

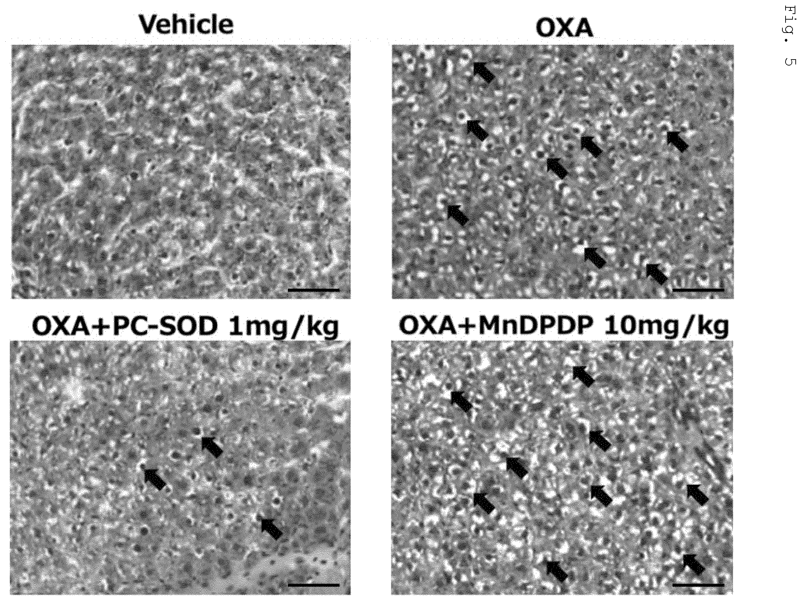

Fig. 5 shows the results of PC-SOD administration against pathological damage to the liver caused by OXA and differentiation from mangafodipir. In the OXA administration group, pathological damage to the liver was observed as compared with the control group (arrows, necrosis of liver cells). Meanwhile, a suppressive effect on necrosis of liver cells caused by OXA was shown in the OXA + PC-SOD 1 mg/kg administration group.

In contrast, the suppressive effect on necrosis of liver cells caused by OXA was not observed in the mangafodipir group.

It should be noted that mangafodipir is an agent similar to calmangafodipir under development as a drug targeting CIPN and was used as a comparator in this example because it was available as a reagent.

From the above results, it was found that PC-SOD had a prophylactic effect on a liver disorder caused by OXA. It was also confirmed that PC-SOD had a prophylactic effect of alleviating a liver disorder, which is one of serious adverse reactions of OXA, as compared with mangafodipir.

### [Test Example 6] Influence of PC-SOD on anti-tumor effect of oxaliplatin

### "Summary"

The influence of the present invention on the anti-tumor effect of an anticancer agent can be determined by observing changes in proliferation of cancer cells caused by oxaliplatin by the method described below.

### "Method"

### (1) Cell culture

Large intestine cancer cells (colo320, HCT116) were added to RPM1640 medium (Thermo Fisher Scientific Inc.) containing 10% fetal bovine serum and 100 units/mL penicillin-streptomycin (Gibco BRL) and cultured at 37°C in a 5% CO₂ incubator.

### (2) Treatment of drugs and measurement of cell proliferation

Cells were seeded on a 96-well plate at 10 000 cells/well and treated with a test solution 24 hours later. A test solution containing OXA alone was diluted 3-fold from 100 µM (10 levels) and added to a 96-well plate. A test solution containing OXA + PC-SOD was obtained by adding the same concentrations of OXA to PC-SOD (50 µg/mL) (n = 3 per group). After cells were cultured for 72 hours, a CCK-8 solution was added in a volume of 1/10 of the solution in each well, and the mixture was measured at 490 nm of absorbance (Tecan, Tecan Japan). Data were expressed with mean ± standard error (SEM). It should be noted that experiment conditions were allowed to be changed as required.

### "Results and interpretation"

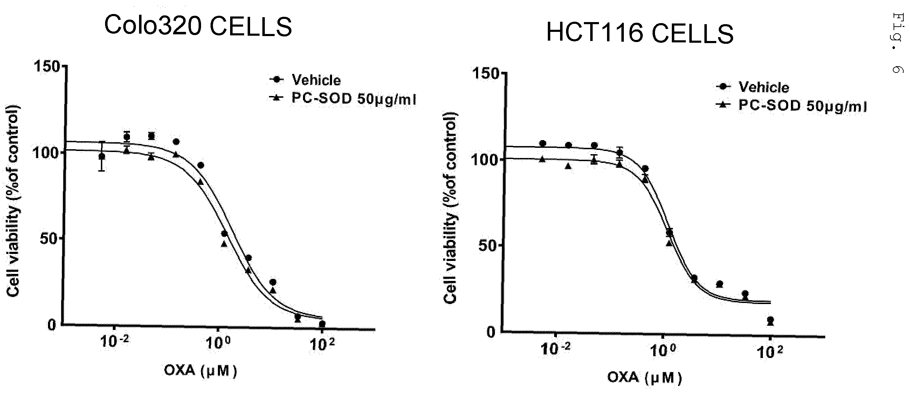

Suppression of proliferation of large intestine cancer cells caused by OXA was confirmed. Given that cell proliferation was suppressed in the OXA + PC-SOD group to the same extent as observed in the OXA alone administration group, it was confirmed that the present invention did not affect the anti-tumor effect of oxaliplatin (Fig. 6).

### [Test Example 7] Neuroprotective effect of PC-SOD in neurite disorder caused by an anticancer agent

### "Summary"

Rat adrenal gland pheochromocytoma-derived PC12 cells are known to be differentiated into neuron-like cells having neurites by nerve growth factor (NGF) and are widely used as a nerve cell model. To assess the neurite-protective action of PC-SOD, the effect of PC-SOD in neurite disorder caused by various anticancer agents was analyzed by using PC12 cells differentiated by NGF.

### [Test Example 7-1] Neuroprotective effect of PC-SOD in a neurite disorder caused by a platinum-based drug (oxaliplatin or cisplatin)

### "Method"

PC12 cells were seeded on a 24-well plate coated with collagen at 5 000 cells/well. The medium was replaced with a differentiation medium (RPMI-1640 medium containing 100 ng/mL NGF and 0.5% FBS) 24 hours later, and cells were cultured for further 96 hours. After neurite elongation, cells were cultured by using association of differentiation media each containing oxaliplatin alone (with a final concentration of 20 µM), cisplatin (abbreviated as CP in the figure of results) alone (with a final concentration of 33 µM), and oxaliplatin or cisplatin and PC-SOD (with final concentrations of 10, 50, and 100 µg/mL) for 24 hours. The images of cells were captured by using a microscope (BZ-X800, Keyence Corporation), and the neurite lengths were measured by using an image analysis software ImageJ (NIH). Thirty cells per well were analyzed in triplicate for each group. The graph shows the mean value of each group, and multiple comparison was performed by Tukey's test (*: p < 0.05).

### "Results"

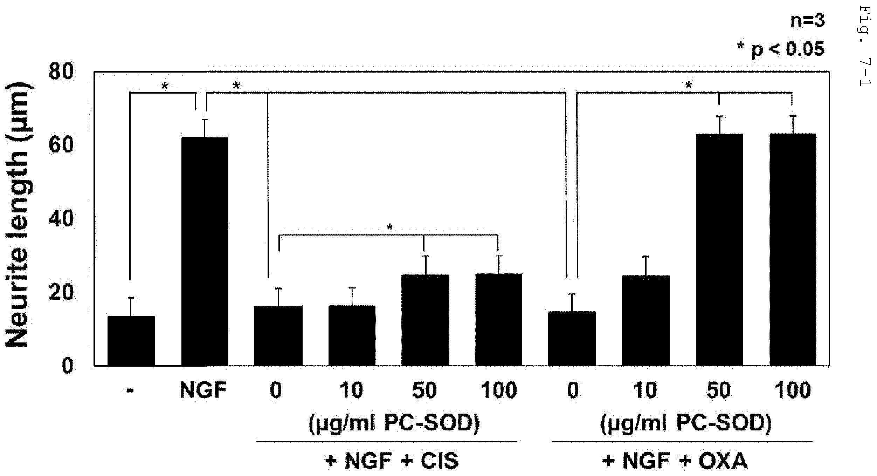

While neurites were retracted by addition of oxaliplatin, the neurite retraction was markedly suppressed by simultaneous addition of PC-SOD dependently on the PC-SOD concentration to the same levels as the level observed in the absence of oxaliplatin. Neurite retraction caused by cisplatin was significantly suppressed as compared with addition of cisplatin alone. These results indicated that PC-SOD protected neurites from neurite damage caused by oxaliplatin and cisplatin. Additionally, it was also indicated that the suppressive action of PC-SOD on neurite retraction was stronger against neurite retraction caused by oxaliplatin than against neurite retraction caused by cisplatin (Fig. 7-1) .

### [Test Example 7-2] Neuroprotective effect of PC-SOD in neurite disorder caused by carboplatin

### "Method"

PC12 cells were seeded on a 24-well plate coated with collagen at 5 000 cells/well. The medium was replaced with a differentiation medium (RPMI-1640 medium containing 100 ng/mL NGF and 0.5% FBS) 24 hours later, and cells were cultured for further 96 hours. After neurite elongation, cells were cultured by using differentiation media each containing carboplatin (with a final concentration of 50 µM) alone or carboplatin and PC-SOD (with final concentrations of 10, 20, 50, and 100 µg/mL) for 24 hours. The images of cells were captured by using a microscope (BZ-X800, Keyence Corporation), and the neurite lengths were measured by using an image analysis software ImageJ (NIH). Thirty cells per well were analyzed in triplicate for each group. The graph shows the mean value of each group, and multiple comparison was performed by Tukey's test (*: p < 0.05).

### "Results and interpretation"

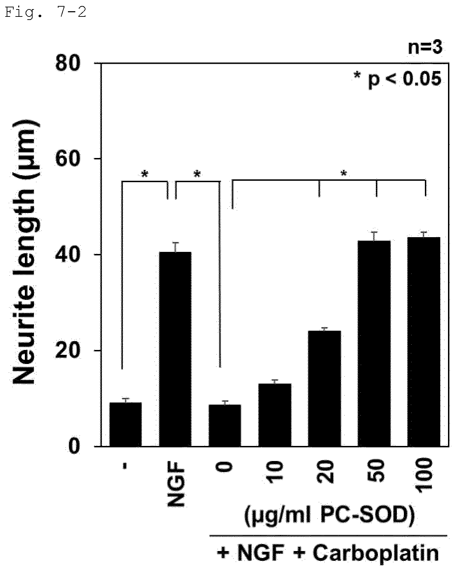

While neurites were retracted by addition of carboplatin, the neurite retraction was suppressed by simultaneous addition of PC-SOD dependently on the PC-SOD concentration. It was indicated that PC-SOD protected neurites against neurite damage caused by carboplatin (Fig. 7-2).

### [Test Example 7-3] Neuroprotective effect of PC-SOD in neurite disorder caused by paclitaxel

### "Method"

PC12 cells were seeded on a 24-well plate coated with collagen at 5 000 cells/well. The medium was replaced with a differentiation medium (RPMI-1640 medium containing 100 ng/mL NGF and 0.5% FBS) 24 hours later, and cells were cultured for further 96 hours. After neurite elongation, cells were cultured by using differentiation media each containing paclitaxel (abbreviated as PTX in the figure of results) alone (with a final concentration of 0.1 µM) or paclitaxel and PC-SOD (with final concentrations of 10, 50, and 100 µg/mL) for 24 hours. The images of cells were captured by using a microscope (BZ-X800, Keyence Corporation), and the neurite lengths were measured by using an image analysis software ImageJ (NIH). Thirty cells per well were analyzed in triplicate for each group. The graph shows the mean value of each group, and multiple comparison was performed by Tukey's test (*: p < 0.05).

### "Results"

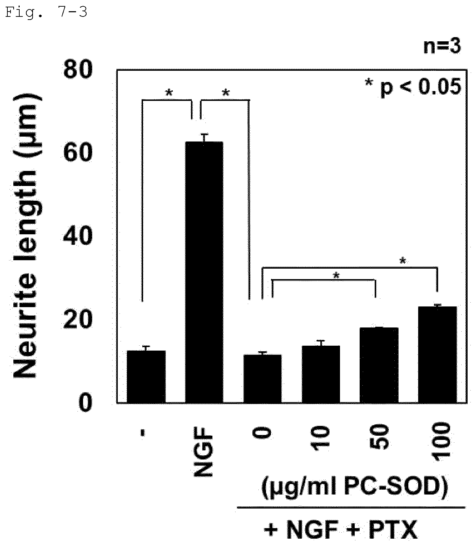

While neurites were retracted by addition of paclitaxel, the neurite retraction was suppressed by simultaneous addition of PC-SOD dependently on the PC-SOD concentration. It was indicated that PC-SOD also protected neurites from neurite damage caused by paclitaxel (Fig. 7-3).

### [Test Example 8] Comparison between PC-SOD and mangafodipir for neuroprotective effect in neurite disorder caused by oxaliplatin

### "Summary"

By using PC12 cells, the neuroprotective effect was compared between PC-SOD and mangafodipir, which has an SOD-like activity, in neurite disorder caused by oxaliplatin.

### "Method"

PC12 cells were seeded on a 24-well plate coated with collagen at 5 000 cells/well. The medium was replaced with a differentiation medium (RPMI-1640 medium containing 100 ng/mL NGF and 0.5% FBS) 24 hours later, and cells were cultured for further 96 hours. After neurite elongation, cells were cultured in differentiation media each containing oxaliplatin alone (with a final concentration of 20 µM) or oxaliplatin and PC-SOD (with a final concentration of 50 µg/mL) or mangafodipir (with final concentrations of 1, 5, 10, 20, and 50 µg/mL) for 24 hours. The images of cells were captured by using a microscope (BZ-X800, Keyence Corporation), and the neurite lengths were measured by using an image analysis software ImageJ (NIH). Thirty cells per well were analyzed in triplicate for each group. The graph shows the mean value of each group, and multiple comparison was performed by Tukey's test (*: p < 0.05) .

### "Results and interpretation"

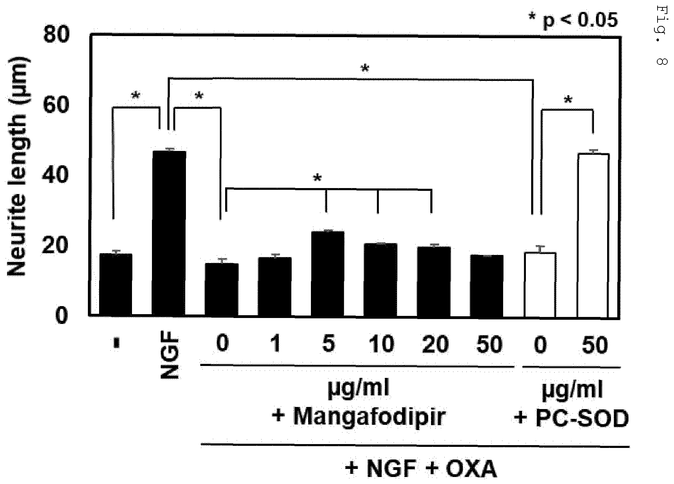

Neurite retraction caused by addition of oxaliplatin was suppressed almost completely by simultaneous addition of 50 µg/mL PC-SOD. In contrast, a weak suppressing tendency was observed after simultaneous addition of mangafodipir at a concentration of 5 µg/mL, and no higher retraction suppressing effect was observed after addition thereof at higher concentrations. These results indicated that PC-SOD had a stronger neuroprotective effect than mangafodipir (Fig. 8).

### [Test Example 9] Comparison between PC-SOD and mangafodipir for cytotoxic concentrations

### "Summary"

By using PC12 cells differentiated by NGF, the concentrations at which cytotoxicity was induced by PC-SOD and mangafodipir were investigated in the presence or absence of oxaliplatin.

### "Method"

PC12 cells were seeded in a 96-well plate coated with collagen at 10 000 cells/well. The medium was replaced with a differentiation medium (RPMI-1640 medium containing 100 ng/mL NGF and 0.5% FBS) 24 hours later, and cells were cultured for further 72 hours. PC-SOD (with final concentrations of 10, 50, 100, 200, and 500 µg/mL) or mangafodipir (with final concentrations of 1, 5, 10, 20, and 50 µg/mL) was added in the presence or absence of oxaliplatin (with a final concentration of 20 µM), and cells were cultured for 24 hours. The cell viability was analyzed by a neutral red assay (*: p < 0.05). Thirty cells per well were analyzed in triplicate for each group. The graph shows the mean value of each group, and multiple comparison was performed by Tukey's test (*: p < 0.05).

### "Results and interpretation"

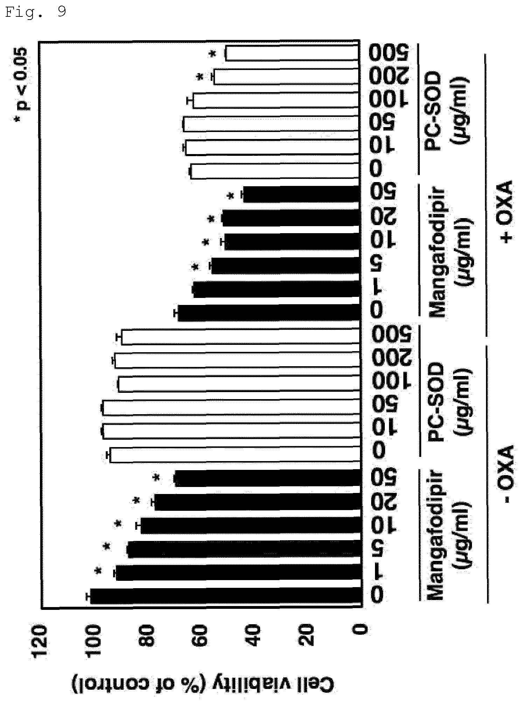

It was shown that addition of PC-SOD did not affect the cell viability at concentrations at which the neuroprotective effect was observed in both the presence and absence of oxaliplatin, and that there was a difference between the concentration at which drug efficacy was observed and the concentration at which cytotoxicity occurred. In contrast, it became evident that addition of mangafodipir decreased the cell viability in a concentration-dependent manner, and that cytotoxicity occurred at concentrations lower than the concentration at which a weak neuroprotective effect was observed. These results indicated that PC-SOD was less cytotoxic and safer than mangafodipir (Fig. 9).

### [Test Example 10] Assessment of a kidney disorder caused by cisplatin

### "Summary"

The effect of the present invention on a kidney disorder attributed to an anticancer agent can be determined by measuring changes in morphology and weight of the kidney caused by cisplatin administration by the method described below.

### "Method"

Six to eight-week-old male SD rats (from 200 to 400 g) were used. Cisplatin (hereinafter may be abbreviated as CIS) was administered into the peritoneal cavity of rats at a dose of 3 mg/kg. A total of eight doses were given with a regimen of twice weekly for four weeks (Days 1, 2, 7, 8, 13, 14, 19, and 20). A solvent for CIS, i.e., physiological saline, was similarly administered to a control group. Cisplatin Injection 50 mg (Nichi-Iko Pharmaceutical Co., Ltd.) was used as CIS. Laboratory animals were divided into three groups: a control group, a CIS administration group, and a group receiving administration of CIS and PC-SOD 1 mg/kg (CIS + PC-SOD administration group) (n = 6 per group).

For an experiment of prevention, PC-SOD was administered into the caudal vein from five to 15 minutes after CIS administration in the CIS + PC-SOD administration group. A total of eight doses were given with a regimen of twice weekly for four weeks (Days 1, 2, 7, 8, 13, 14, 19, and 20). A solvent for PC-SOD was similarly administered to the control group and the CIS administration group. The rat kidney weight was measured, and changes in the kidney weight were corrected with body weight. Further, a photograph of the kidney was taken to check changes in size and morphology of the kidney (Scale bar = 10 mm).

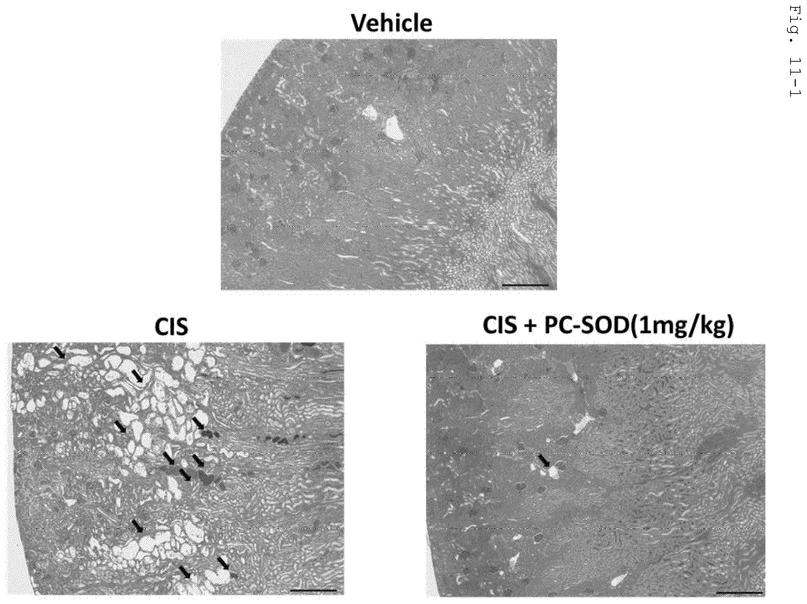

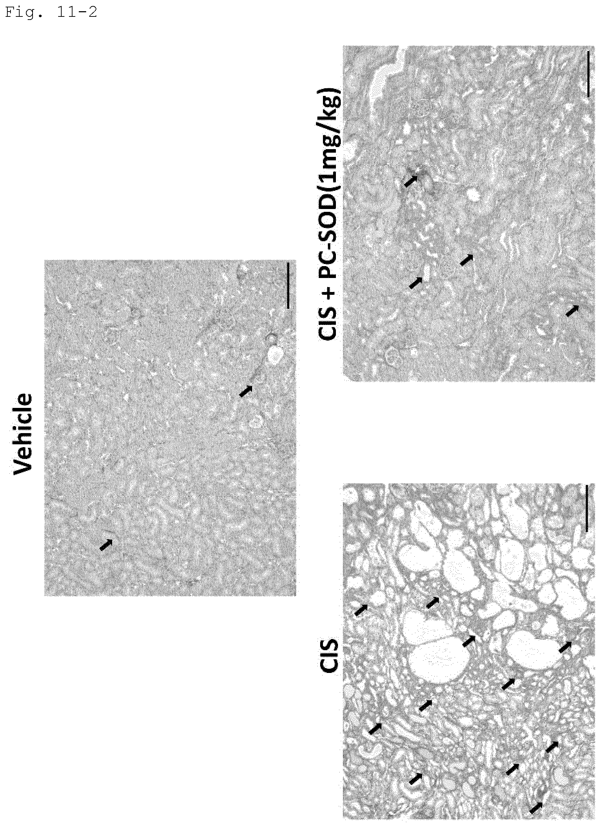

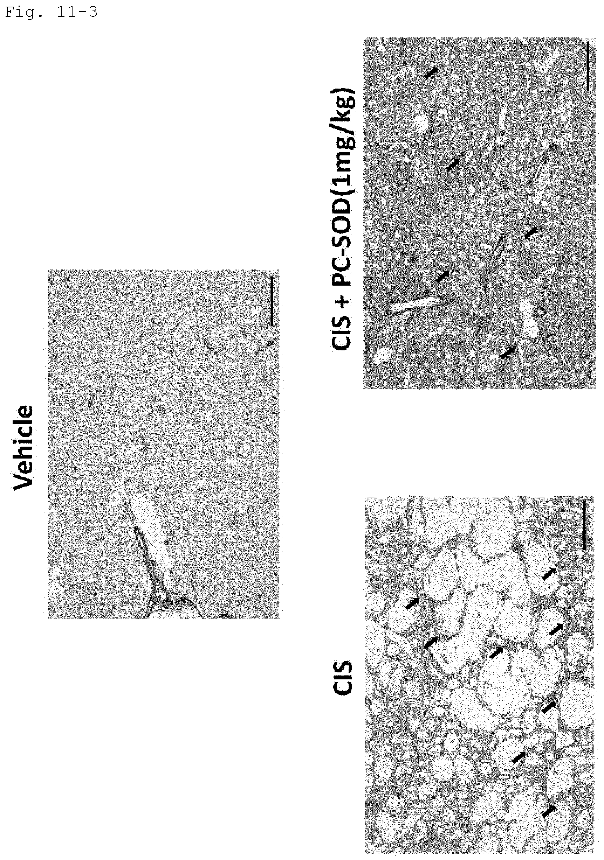

For pathological analysis, a split was put in the sagittal plane of the collected kidney, and the kidney was fixed with 10% formalin to prepare a paraffin block by using Histos 5 (Milestone) and an embedding center (EG1160, Leica). The sample block was thinly sliced by using a microtome (MR2245, Leica) at a thickness of 3 µm to prepare a slide. The prepared slide was subjected to Periodic acid-Schiff (PAS) staining for assessment of clinical condition and Picrosirius Red staining for assessment of fibrosis. A fibrosis marker α-SMA was stained by an immunostaining method. The slides were observed at 40 and 100-fold magnifications by using the optical microscope function of a microscope (BZ-X800, Keyence Corporation). Observation and assessment were performed by observing the whole section. Four to nine images of tissue on the cortical side on which the disorder was induced were randomly taken up, and inflammation, tubular necrosis, protein agglutination in renal tubules, vacuolation, and necrosis were assessed for changes in clinical condition. Fibrin on the cortical side or antibody-positive regions were observed for assessment of fibrosis. (Scale bar = 500 µm (Fig. 11-1), Scale bar = 200 µm (Figs. 11-2 and 11-3)).

### "Results and interpretation"

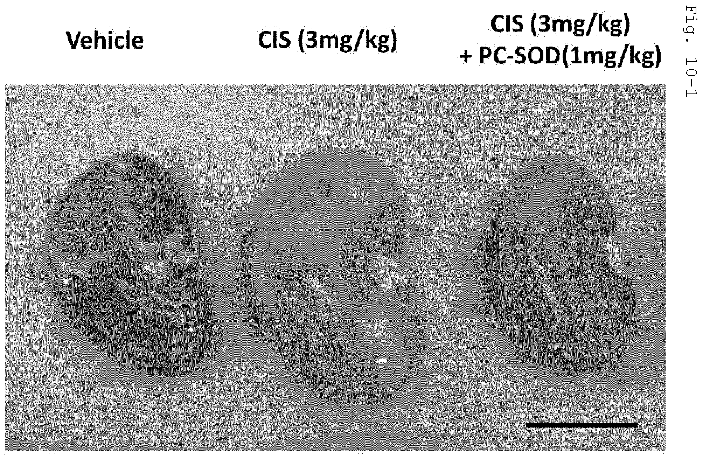

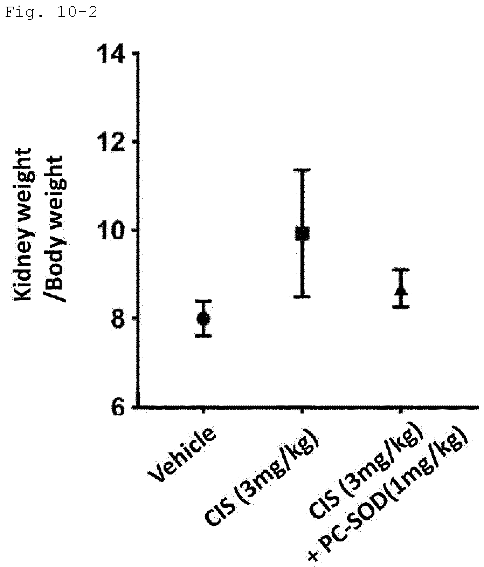

The effect of PC-SOD on a kidney disorder caused by CIS was assessed by using a change in the kidney weight as a measure. The results are shown in Figs. 10-1 and 10-2. Fig. 10-1 shows the photograph of the kidney for each group. In the CIS administration group, weight gain and swelling of the kidney were observed as compared with the control group. In contrast, the suppressive effect on an increase in the kidney weight and swelling of the kidney caused by CIS was confirmed in the CIS +PC-SOD 1 mg/kg administration group (Fig. 10-2).

Fig. 11-1 shows the results of PC-SOD administration against pathological damage to the kidney caused by CIS. In the CIS administration group, pathological damage phenomena, such as vacuolation, protein agglutination in renal tubules, and inflammatory cell agglutination, were observed (arrows; vacuolization, protein agglutination in renal tubules, infiltration of inflammatory cells, and the like) as compared with the control group. In contrast, a suppressive effect on pathological damage to the kidney caused by CIS was observed in the CIS + PC-SOD 1 mg/kg administration group. Figs. 11-2 and 11-3 show the results of PC-SOD administration against renal fibrosis caused by CIS. The results of Picrosirius Red staining and α-SMA staining showed that fibrin was highly expressed in interstitial parts of renal tubules in the CIS administration group as compared with the control group (arrows pint to fibrosis and fibrin-positive regions). In contrast, decreases of fibrosis-positive regions in the kidney caused by CIS were detected in the group receiving administration of CIS + PC-SOD 1 mg/kg.

From the above results, it was found that PC-SOD had a suppressive effect on swelling and fibrosis of the kidney caused by CIS, confirming that PC-SOD has a prophylactic effect that alleviates a kidney disorder, which is one of serious adverse drug reactions of CIS.

In particular, since swelling of the kidney associated with administration of an anticancer agent is closely related to fluid retention due to oliguria, PC-SOD is effective for a kidney disorder associated with administration of an anticancer agent, in particular, nephropathy with oliguria.

In this test, cisplatin was administered twice weekly for four weeks. Therefore, it is considered that cisplatin-induced nephropathy occurred in this test system. In general, an acute kidney disorder is a kidney disorder which occurs during a period from several hours to several days, and a chronic kidney disorder (chronic kidney disease) is a condition in which a "kidney disorder" or "decreased renal function" persists for three months or longer. Therefore, it was indicated that PC-SOD could have a suppressive effect on swelling and fibrosis of the kidney in a transitional phase from an acute kidney disorder to a chronic kidney disorder, preferably a transitional phase from acute cisplatin-induced nephropathy to chronic cisplatin-induced nephropathy.

### [Test Example 11] Effect of PC-SOD on toxicity of an anticancer agent against RAW cells

### "Summary"

Anticancer agents have toxicity against RAW cells. Whether PC-SOD exhibits an inhibitory action on the cytotoxicity was investigated.

### "Method"

RAW cells were seeded on a 96-well plate at 20 000 cells/well (D-MEM high glucose medium containing 10% inactivated FBS). Gemcitabine alone, oxaliplatin alone, cisplatin alone, carboplatin alone, gemcitabine and PC-SOD, oxaliplatin and PC-SOD, cisplatin and PC-SOD, or carboplatin and PC-SOD were added 24 hours later. The number of viable cells was counted 24 hours later by measuring chemical luminescence by using CellTiter-Glo^{™} 2.0 Cell Viability Assay (Promega) and a plate reader (Tecan, Infinite M Plex). Cells for four wells were analyzed for each group. The graph shows the mean value of each group, and multiple comparison was performed by Dunnett's test (*, p < 0.05; **, p < 0.01 vs control; #, p < 0.05; ##, p < 0.01 vs anticancer agent alone).

### "Results and assessment"

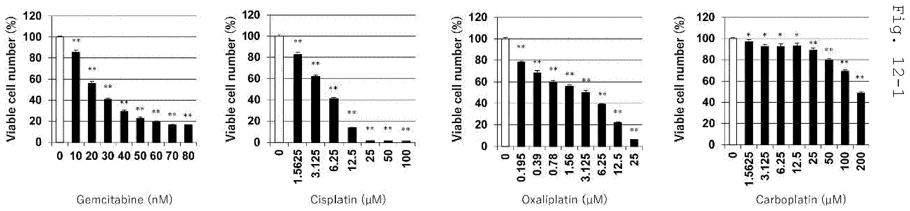

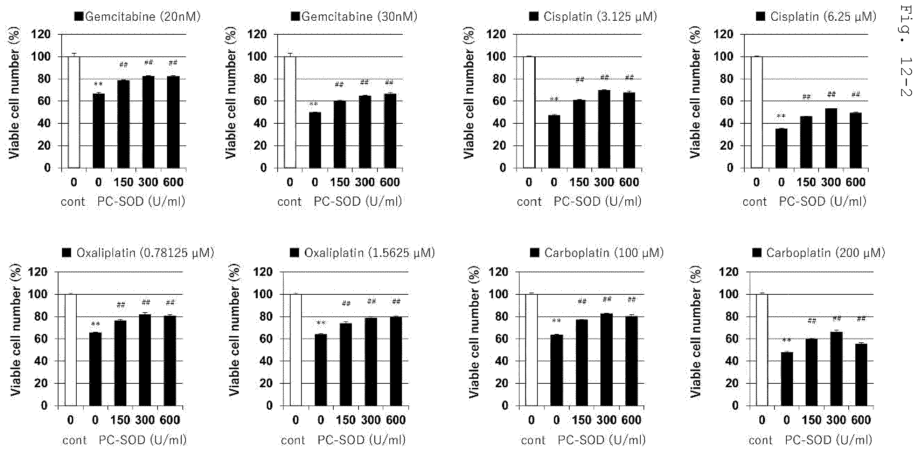

It was observed that treatment with gemcitabine alone, oxaliplatin alone, cisplatin alone, and carboplatin alone had decreased the number of viable cells dependently on the concentration of the anticancer agent (Fig. 12-1). In contrast, simultaneous addition of PC-SOD significantly suppressed the anticancer agent-dependent decreases in the number of viable cells dependently on the concentration of PC-SOD. These results indicated that PC-SOD protected blood cells from cytotoxicity of an anticancer agent (Fig. 12-2).

### [Test Example 12] Effect of PC-SOD on active oxygen production in RAW cells caused by anticancer agent

### "Summary"

Anticancer agents promote active oxygen production in RAW cells. Whether PC-SOD exhibits an inhibitory action on the active oxygen production was investigated.

### "Method"

RAW cells were seeded on a 96-well plate for fluorescence at 20 000 cells/well (D-MEM high glucose medium containing 10% inactivated FBS). Gemcitabine alone, oxaliplatin alone, cisplatin alone, carboplatin alone, gemcitabine and PC-SOD, oxaliplatin and PC-SOD, cisplatin and PC-SOD, or carboplatin and PC-SOD were added 24 hours later. Sixteen hours later, cells were treated with an HBSS solution containing dissolved MitoSOX^{™} Red Mitochondrial Superoxide Indicator (Invitrogen^{™}) for 30 minutes, and the medium was replaced with D-MEM high glucose medium containing 1% inactivated FBS. Active oxygen production in mitochondria was determined by measuring fluorescence (Ex 510 nm, Em 580 nm) by using a plate reader (Infinite M Plex, Tecan). Cells for four wells were analyzed for each group. The graph shows the mean value of each group, and multiple comparison was performed by Dunnett's test (*, p < 0.05; **, p < 0.01 vs control; #, p < 0.05; ##, p < 0.01 vs anticancer agent alone).

### "Results and assessment"

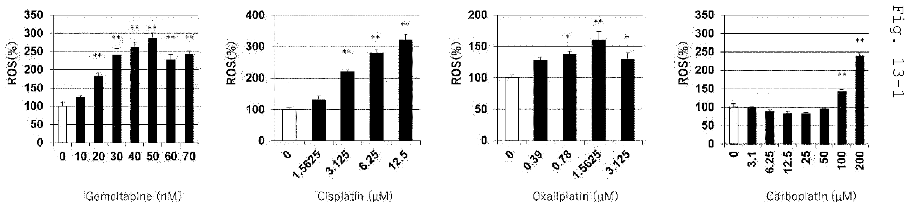

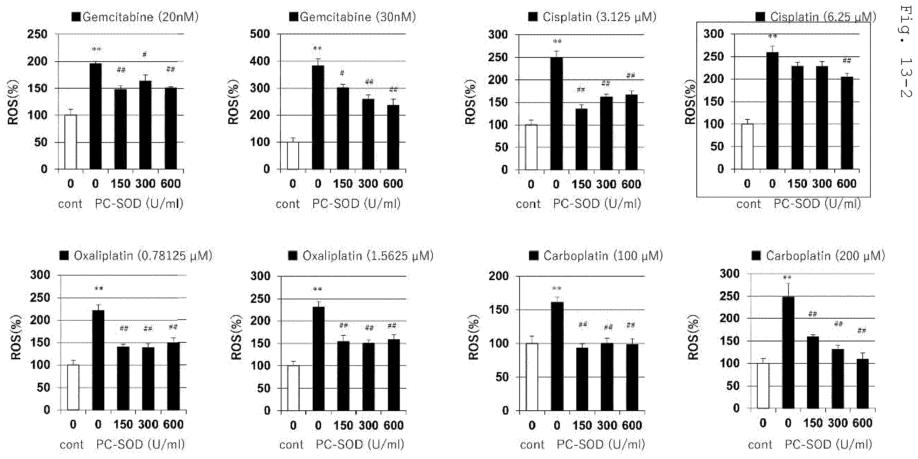

It was observed that treatment with gemcitabine alone, oxaliplatin alone, cisplatin alone, or carboplatin alone had induced active oxygen production dependently on the concentration of the anticancer agent (Fig. 13-1). In contrast, simultaneous addition of PC-SOD significantly suppressed the anticancer agent-dependent production of active oxygen dependently on the concentration of PC-SOD (Fig. 13-2). These results indicated that PC-SOD suppressed active oxygen production in mitochondria caused by an anticancer agent. Additionally, discussion along with the results of the analysis of the number of viable cells indicated that PC-SOD protected blood cells from cytotoxicity of an anticancer agent via an antioxidative action (Fig. 13).

### [Test Example 13] Assessment of myelosuppression caused by cisplatin

### "Summary"

The effect of the present invention on myelosuppression attributed to an anticancer agent can be determined by analyzing changes in peripheral blood cells caused by cisplatin administration by the method described below.

### "Method"

Six to eight-week-old male SD rats (from 200 to 400 g) were used. Cisplatin (hereinafter may be abbreviated as CIS) was administered into the peritoneal cavity of rats at a dose of 3 mg/kg. A total of eight doses were given with a regimen of twice weekly for four weeks (Days 1, 2, 7, 8, 13, 14, 19, and 20). A solvent for CIS, i.e., physiological saline, was similarly administered to a control group. Cisplatin Injection 50 mg (Nichi-Iko Pharmaceutical Co., Ltd.) was used as CIS. Laboratory animals were divided into three groups: a control group, a CIS administration group, and a group receiving administration of CIS and PC-SOD 1 mg/kg (CIS + PC-SOD administration group) (n = 6 per group). For an experiment of prevention, PC-SOD was administered into the caudal vein from five to 15 minutes after CIS administration in the CIS + PC-SOD administration group. A total of eight doses were given with a regimen of twice weekly for four weeks, (Days 1, 2, 7, 8, 13, 14, 19, and 20). A solvent for PC-SOD was similarly administered to the control group and the CIS administration group. Peripheral blood was collected from the rat caudal vein three days later, and peripheral blood was analyzed by using an automated blood cell counter (MEK-6458, Nihon Kohden Corporation).

### "Results and interpretation"

White blood cells (WBCs) and platelets (PLTs) were decreased by CIS administration, but decreases in WBCs and PLTs were suppressed in the PC-SOD simultaneous administration group.

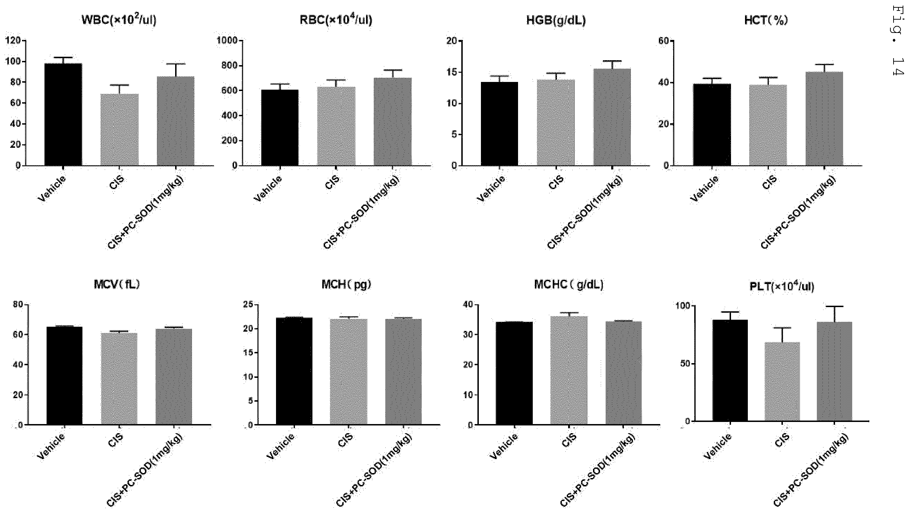

The above results indicated that PC-SOD protected against myelosuppression caused by CIS (Fig. 14).

### [Test Example 14] Identification of PC modification sites in PC-SOD

PC-SOD is typically manufactured by the method described in Patent Literature 14, 15, or 16, more preferably the method described in Patent Literature 16. As understood from these literatures, since PC is allowed to bind to SOD in the presence of a solvent in the manufacture of PC-SOD, the same sites of amino acid residues in SOD to which PC binds cannot be strictly identified for all SOD molecules in a practical manner. Therefore, the PC-SOD of the present invention is a heterogenous aggregate composed of PC-SOD molecules in which PC binds to a SOD molecule in various numbers and at various binding sites.

Therefore, the structure of "PC-SOD," in particular, PC binding sites and the number of PC molecules binding to a PC-SOD molecule can be described with maximal clarity by specifying amino acid residues in an SOD molecule to which PC molecules can bind and binding rates thereof in a certain range.

From the above-mentioned viewpoints, it was considered that analyzing amino acid residues in an SOD molecule to which PC molecules actually bind and binding rates thereof was the best means to determine the structure of PC-SOD.

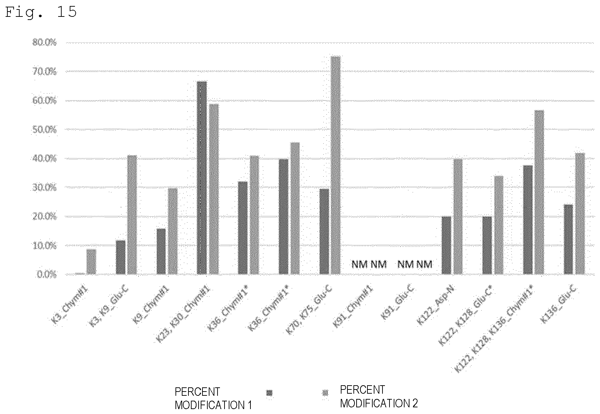

Therefore, to identify the PC modification sites in PC-SOD, enzymatic digestion analysis and LC/MS analysis were performed for PC-SOD. Fig. 15 shows the obtained PC modification sites and PC modification rates in PC-SOD. It should be noted that the number after a one-letter amino acid code represents the number of residues from the N terminus of an SOD molecule.

The above analysis was performed twice, and the mean values of the results are shown in the following table.

**[Table 1]**

| SOD modification site | Mean percent modification |
|---|---|
| K3, N terminus, or T2 | 5% |
| K9 | 23% |
| K23 | 63% |
| K36 | 43% |
| K70 or K75 | 53% |
| K91 | 0% |
| K122 or K128 | 27% |
| K136 | 33% |

These results indicated that there were amino acid residues in an SOD molecule to which PC preferentially binds with selectivity or tropism, and that there were amino acid residues in an SOD molecule to which PC does not bind.

## Claims

1. A pharmaceutical composition comprising a lecithinized superoxide dismutase, for treating or preventing a disorder associated with administration of an anticancer agent.

2. The pharmaceutical composition according to claim 1, wherein the disorder associated with administration of an anticancer agent is a disorder selected from the group consisting of a neurological disorder, a liver disorder, drug-induced deafness, a kidney disorder, myelosuppression, and a combination of one or more thereof.

3. The pharmaceutical composition according to claim 2, wherein the neurological disorder is a peripheral neurological disorder, neuropathy, neuropathic pain, allodynia, hyperalgesia or hypoalgesia, or thermal sensation disorder.

4. The pharmaceutical composition according to claim 2, wherein the neurological disorder is a neurological disorder resulting from decreased nerve fiber density, reduced or retracted neurites, or damaged dorsal root ganglion (DRG) neurons.

5. The pharmaceutical composition according to claim 1, wherein the disorder associated with administration of an anticancer agent is chemotherapy-induced peripheral neuropathy (CIPN).

6. The pharmaceutical composition according to claim 1 or 2, wherein the disorder associated with administration of an anticancer agent is a liver disorder, and the anticancer agent is a platinum-based drug.

7. The pharmaceutical composition according to claim 1 or 2, wherein the disorder associated with administration of an anticancer agent is drug-induced deafness, and the anticancer agent is a platinum-based drug.

8. The pharmaceutical composition according to claim 1 or 2, wherein the disorder associated with administration of an anticancer agent is a kidney disorder, and the anticancer agent is a platinum-based drug.

9. The pharmaceutical composition according to claim 8, wherein the kidney disorder is cisplatin-induced nephropathy, and the anticancer agent is cisplatin.

10. The pharmaceutical composition according to claim 8 or 9, wherein the kidney disorder is nephropathy with oliguria.

11. The pharmaceutical composition according to any one of claims 8 to 10, wherein the kidney disorder is in a transitional phase from an acute kidney disorder to a chronic kidney disorder.

12. The pharmaceutical composition according to claim 1 or 2, wherein the disorder associated with administration of an anticancer agent is myelosuppression, and the anticancer agent is a platinum-based drug or a metabolism antagonist.

13. The pharmaceutical composition according to any one of claims 1 to 11, wherein the anticancer agent is at least one selected from the group consisting of a platinum-based drug, a metabolism antagonist, a taxane drug, a vinca alkaloid drug, and a proteasome inhibitor.

14. The pharmaceutical composition according to claim 13, wherein the anticancer agent is a platinum-based drug.

15. The pharmaceutical composition according to claim 13 or 14, wherein the anticancer agent is oxaliplatin or cisplatin.

16. The pharmaceutical composition according to any one of claims 13 to 15, wherein the anticancer agent is administered in FOLFOX therapy or XELOX therapy (CapeOX therapy).

17. The pharmaceutical composition according to any one of claims 1 to 16, wherein the anticancer agent is administered to a cancer patient suffering from one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, chorionic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer.

18. The pharmaceutical composition according to any one of claims 1 to 16, wherein the anticancer agent is administered to a patient who has a medical history of one or more cancers selected from the group consisting of ovarian cancer, non-small cell lung cancer, breast cancer, endometrial cancer, head and neck cancer, esophageal cancer, leukemia, malignant lymphoma, pediatric tumor, multiple myeloma, malignant astrocytoma, neuroglioma, chorionic disease, germ cell tumor, lung cancer, testicular tumor, bladder cancer, renal pelvic tumor, urethral tumor, prostate cancer, uterine cervical cancer, neuroblastic tumor, small-cell lung cancer, bone sarcoma, malignant pleural mesothelioma, malignant bone tumor, kidney cancer, penis cancer, each bone and soft tissue tumor, liver cancer, thyroid gland cancer, retroperitoneal tumor, bone metastasis, testicular cancer, gallbladder cancer, biliary tract cancer, biliary duct cancer, adrenal cancer, neuroblastoma, hepatoblastoma, primary hepatic malignant tumor, medulloblastoma, stomach cancer, pancreas cancer, urothelial cancer, extragonadal tumor, Langerhans cell histiocytosis, mantle cell lymphoma, lymphoplasmacytic lymphoma, small intestine cancer, colon cancer, rectal cancer, and large intestine cancer and from whom the cancer or cancers were removed.

19. The pharmaceutical composition according to any one of claims 1 to 18, which is administered in combination with an anticancer agent.

20. The pharmaceutical composition according to any one of claims 1 to 19, wherein the lecithinized superoxide dismutase is a lyophilized formulation.

21. The pharmaceutical composition according to any one of claims 1 to 20, comprising a lecithinized superoxide dismutase,
the lecithinized superoxide dismutase comprising a lecithinized superoxide dismutase in which at least one amino group in a human-derived superoxide dismutase subunit coordinated with copper and/or zinc and having a mercapto group in cysteine at position 111 replaced by a hydroxyethylthio group is modified with a lecithin residue of the following formula (I): 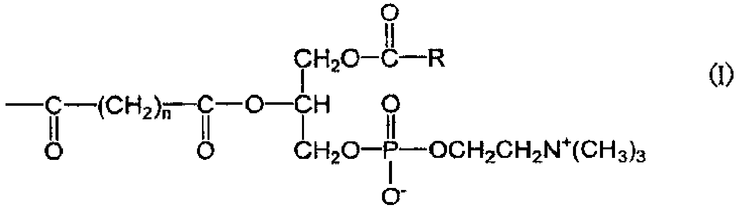 [wherein R represents an alkyl group with 8 to 30 carbon atoms, and n is an integer of from 2 to 10] directly or via a linker, and
the lecithinized superoxide dismutase comprises a lecithinized superoxide dismutase (A), as a principal component, constituted by lecithinized superoxide dismutase subunits each of which independently has m number of amino groups [m is an integer of from 1 to 4, and the mean value of m in lecithinized superoxide dismutase subunits is from 1.5 to 2.4] substituted with the lecithin residue, wherein
the lecithinized superoxide dismutase (A) is constituted by the following lecithinized superoxide dismutase subunits:
from 25 to 40 mol% lecithinized superoxide dismutase subunit (a1) with m = 1,
from 35 to 50 mol% lecithinized superoxide dismutase subunit (a2) with m = 2,
from 10 to 20 mol% lecithinized superoxide dismutase subunit (a3) with m = 3, and
from 5 to 15 mol% lecithinized superoxide dismutase subunit (a4) with m = 4.

22. The pharmaceutical composition according to any one of claims 1 to 21, wherein PC bind to SOD with the following binding rates in the lecithinized superoxide dismutase:
(1) the binding rate of the PC at the binding sites of K3, N terminus, and T2 is from 1% to 10% as the sum of these amino acid residues;
(2) the binding rate of the PC at the binding site of K9 is from 10% to 30%;
(3) the binding rate of the PC at the binding site of K23 is from 50% to 75%;
(4) the binding rate of the PC at the binding site of K36 is from 30% to 55%;
(5) the binding rate of the PC at the binding site of K70 or K75 is from 10% to 80% as the sum of these amino acid residues;
(6) the binding rate of the PC at the binding site of K122 or K128 is from 10% to 45% as the sum of these amino acid residues;
(7) the binding rate of the PC at binding site of K136 is from 10% to 60%; and
on average 1 to 8 PC molecules are bound in a PC-SOD molecule as the sum of the (1) to (7).

23. The pharmaceutical composition according to any one of claims 1 to 22, wherein a superoxide dismutase in the lecithinized superoxide dismutase has an amino acid sequence set forth in SEQ ID NO: 1.

24. Use of a lecithinized superoxide dismutase for manufacturing a pharmaceutical composition for treating or preventing a disorder associated with administration of an anticancer agent.

25. The use according to claim 24, wherein the disorder associated with administration of an anticancer agent is a disorder selected from the group consisting of a neurological disorder, a liver disorder, drug-induced deafness, a kidney disorder, myelosuppression, and a combination of one or more thereof.

26. A lecithinized superoxide dismutase for use in treating or preventing a disorder associated with administration of an anticancer agent.

27. The lecithinized superoxide dismutase according to claim 26, wherein the disorder associated with administration of an anticancer agent is a disorder selected from the group consisting of a neurological disorder, a liver disorder, drug-induced deafness, a kidney disorder, myelosuppression, and a combination of one or more thereof.

28. A method for treating or preventing a disorder associated with administration of an anticancer agent, comprising administration of a lecithinized superoxide dismutase.

29. The method according to claim 28, wherein the disorder associated with administration of an anticancer agent is a disorder selected from the group consisting of a neurological disorder, a liver disorder, drug-induced deafness, a kidney disorder, myelosuppression, and a combination of one or more thereof.
